(19) Europäisches Patentamt
European Patent Office
Office européen des brevets

(11) EP 2 983 618 B1

(12) # EUROPEAN PATENT SPECIFICATION

(45) Date of publication and mention of the grant of the patent:
**17.04.2019 Bulletin 2019/16**

(21) Application number: **14703422.7**

(22) Date of filing: **09.01.2014**

(51) Int Cl.:
***A61F 2/16*** *(2006.01)*

(86) International application number:
**PCT/IB2014/058142**

(87) International publication number:
**WO 2014/167425 (16.10.2014 Gazette 2014/42)**

(54) **PHAKIC LENS DEVICE WITH OPENINGS AND CONCENTRIC ANNULAR ZONES**

PHAKISCHE LINSENVORRICHTUNG MIT ÖFFNUNGEN UND MIT KONZENTRISCHEN RINGFÖRMIGEN ZONEN

DISPOSITIF DE LENTILLE PHAKIQUE POURVU D'OUVERTURES ET DE ZONES ANNULAIRES CONCENTRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.04.2013 IN 1353MU2013**

(43) Date of publication of application:
**17.02.2016 Bulletin 2016/07**

(73) Proprietor: **Dave, Jagrat Natavar**
**Vadodara, Gujarat 319440 (IN)**

(72) Inventors:
- **ARGAL, Sanjay Ram Swaroop**
  **Vadodara, Gujarat 390020 (IN)**
- **HUSSAIN, Munavvar Tahir**
  **Vadodara, Gujarat 390020 (IN)**

(74) Representative: **Kindermann, Peter**
**Karl-Böhm-Strasse 1**
**85598 Baldham (DE)**

(56) References cited:
**EP-A1- 0 024 126**
**WO-A1-2005/099630**
**WO-A1-2011/024125**
**DE-A1- 10 351 470**
**US-A1- 2005 149 184**
**US-A1- 2005 187 622**
**US-A1- 2007 060 644**
**US-A1- 2008 097 599**
**US-A1- 2008 109 078**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 983 618 B1

Printed by Jouve, 75001 PARIS (FR)

**Description**

FIELD AND BACKGROUND OF THE INVENTION

The present invention, in some embodiments thereof, relates to an implantable lens device and, more particularly, but not exclusively, to a phakic lens device. Some embodiments not part of the present invention relate to a method of fabrication the phakic lens device, and some embodiments not part of the present invention relate to a method of using the phakic lens device.

The human eye is a complex anatomical device, which facilitates interpretation of shapes, colors and dimensions of objects by processing the light they reflect or emit. Similarly to a camera, the eye is able to refract light and produce a focused image that can stimulate neural responses and provide the ability to see.

For the purpose of providing a self-contained document, following is a description of the principle of operation of the mammalian eye, in general, and of the cornea in particular. The iris regulates the amount of light admitted to the interior of the eye, the cornea and the lens focus the light rays from an object being viewed onto the retina which transmits the image of the object to the brain via the optic nerve. About 75% of the focusing is provided by the cornea, with the other 25% provided by the crystalline lens which may acquire variable focal lengths.

The cornea is the most anterior structure of the eye. Since it has to be transparent to allow light to enter the eye, there are no blood vessels in the cornea. The cornea is composed of collagen fibers packed together in an organized pattern, thereby providing the cornea its light transparent nature. The cornea has the highest concentration of nerve endings in the entire body, thus making it extremely sensitive to any kind of trauma.

The front view of the cornea is of an aspheric shape, where the vertical dimension is smaller than the horizontal dimension by about 1-2 %. The anterior is typically about 11.7 mm in diameter.

The optical power of the eye is determined by the optical power of the cornea and crystalline lens. In the normal healthy eye, a sharp image is formed on the retina. In many cases, however, images are either formed in front of the eye because eye is abnormally long (axial myopia) or formed back of the retina because the eye is abnormally short (axial hyperpia). The cornea may also be asymmetric, resulting in an uncompensated cylindrical refractive error referred to as corneal astigmatism. In addition, due to age related reduction in the natural lens accommodation, the eye may become presbyopic resulting in the need of bifocal or multifocal correction device.

Spectacles or contact lenses are required to compensate for the focus of the natural lens or axial length of the eye. Recent technological developments have provided a deformable and relatively permanent artificial intraocular lens (IOL) that can be implanted into the eye to provide permanent vision correction. Phakic IOLs, also known as Implantable phakic contact lenses (IPCLs), are implantable lenses which are used in combination with natural lens in the eye.

IPCL corrects vision in the same way as external contact lenses, except that the IPCL is placed inside the chamber of the eye where it is expected to provide permanent solution to myopia, particularly to refractive errors of above -5.0 D. IPCLs are also similar to the foldable intraocular lenses which are used during cataract surgery to replace the eye's natural lens. However, during the IPCL surgery, the natural lens is kept intact in the eye and it co-works with the implanted lens to correct the refractive error.

The basic concept of phakic IOLs was disclosed in U.S. Pat. No. 4,585,456 to Blackmore. Blackmore describes a phakic lens which is placed on the surface of the natural lens and centered by being held in the ciliary sulcus. Several other corrective lens implants having stiffened or rigid haptics are described in U.S. Patent No. 5,258,025, and U.S. Patent No. 5,078,742. U.S. Patent No. 4,769,035 discloses a phakic intraocular lens which is placed directly on the anterior surface of the natural lens.

Additional background art includes U.S. Patent Nos. 4,769,035, 5,258,025, 6,015,435 and 6,106,553, and International Publication Nos. WO/1995/028897 and WO/2002/003891.

A phakic lens device according to the preamble of claim 1 is known from document US-A-2005/0149184.

SUMMARY OF THE INVENTION

According to some embodiments of the invention the present invention there is provided a lens device. The lens device is structurally adapted to be positioned in the chamber of the eye, preferably the posterior chamber of the eye. The device according to some embodiments of the present invention comprises a generally circular optical section characterized by at least one optical power, two generally flat haptic structures at radially opposite sides of the optical part, and a vaulted section connecting the optical section and the haptic structures.

According to some embodiments of the present invention each haptic structure has at least three outwardly protruding pads for fixating the haptic structures in the ciliary sulcus.

According to some embodiments of the invention the vaulted section comprises at least one opening for allowing flow of liquid, through the vaulted section, between the posterior chamber and the anterior chamber of the eye.

According to some embodiments of the invention the optical section comprises at least one opening for allowing

flow of liquid, through the optical section, between the posterior chamber and the anterior chamber of the eye.

According to some embodiments of the invention the device comprises a transition zone between the optical section and the vaulted section, wherein the transition zone has at least one opening for allowing flow of liquid, through the transition zone, between the posterior chamber and the anterior chamber of the eye.

According to some embodiments of the invention the device is structurally adapted to be positioned below the iris level by its entirety.

According to some embodiments of the invention the at least one opening of the vaulted section has an aspect ratio of less than 2.

According to some embodiments of the invention the at least one opening of the vaulted section is elongated.

According to some embodiments of the invention the at least one opening of the vaulted section is curved.

According to some embodiments of the invention the at least one optical power is refractive.

According to some embodiments of the invention the at least one optical power is diffractive.

According to some embodiments of the invention the optical section is monofocal.

According to some embodiments of the invention the optical section is multifocal.

According to some embodiments of the invention a first optical side of the optical section has an aspheric profile and a diffractive pattern formed thereon, and an opposite optical side of the optical section has a toric profile and is devoid of any diffractive pattern.

According to some embodiments of the invention the toric profile is devoid of spherical aberration.

According to some embodiments of the invention the aspheric profile is characterized by a conic constant in a range of from about -1.1 to about -3.0, inclusive.

According to some embodiments of the invention the aspheric profile is characterized by a conic constant in a range of from about -1.1 to about -1.37, inclusive.

According to some embodiments of the invention a refractive power is substantially uniform across the aspheric profile.

According to some embodiments of the invention an overall effective refractive area of the lens body is less than 60 % of the total effective area of the lens body.

According to some embodiments of the invention the optical section comprises a diffractive pattern having a plurality of concentric annular zones having surfaces separated by slanted steps having surfaces, wherein the surfaces of the concentric zones effect both diffraction and refraction of incident light, while the surfaces of the steps are substantially devoid of any diffractive or refractive power.

According to some embodiments of the invention each step of the steps is characterized by a radius, a slope and a height, and wherein the slope and the height are at least non-increasing functions of the radius.

According to some embodiments of the invention the plurality of concentric zones comprises at least 4 concentric zones. According to some embodiments of the invention the plurality of concentric zones comprises at least 8 concentric zones. According to some embodiments of the invention the plurality of concentric zones comprises at least 20 concentric zones. According to some embodiments of the invention the plurality of concentric zones comprises at most 40 concentric zones. According to some embodiments of the invention the plurality of concentric zones comprises N concentric zones, wherein N is an integer from about 4 to about 40. According to some embodiments of the invention the plurality of concentric zones comprises about 4 concentric zones. According to some embodiments of the invention the plurality of concentric zones comprises about 6 concentric zones. According to some embodiments of the invention the plurality of concentric zones comprises about 8 concentric zones. According to some embodiments of the invention the plurality of concentric zones comprises about 30 concentric zones.

According to some embodiments of the invention each step of the steps has a width which is from about 0.17 microns to about 0.2 microns.

According to some embodiments of the invention the heights are less than 2 $\mu$m.

According to some embodiments of the invention the heights vary from about 1.9 micron at the center to about 0.09 micron at the edge.

According to some embodiments of the invention the slopes vary from about 84° at the center to about 25° at the edge.

According to some embodiments of the invention at least one zone of the plurality of zones has a diffraction pattern formed thereon, such that a diffractive power the zone gradually decreases thereacross.

According to some embodiments of the invention the zones are substantially equal in area.

According to some embodiments of the invention the zones and the steps transmit at least 80 % of incident light.

According to some embodiments of the invention at least one of the optical section, the haptic structures and the vaulted section, comprises at least one substance selected from the group consisting of PMMA, HEMA, collagen and acrylic material.

According to some embodiments of the invention at least one of the optical section, the haptic structures and the vaulted section, comprises a hydrophilic material.

According to some embodiments of the invention at least one of the optical section, the haptic structures and the vaulted section, comprises a hydrophobic material.

According to some embodiments of the invention at least one of the optical section, the haptic structures and the vaulted section, comprises a polymeric or co-polymeric composition, and at least one curcuminoid compound incorporated in or on the polymeric or co-polymeric composition and/or the lens body.

According to some embodiments of the invention at least one of the optical section, the haptic structures and the vaulted section, comprises a co-polymeric composition having a polymeric backbone composed of a plurality of backbone units covalently linked to one another, the backbone units being derived from a pre-polymerization mixture of monomers.

According to some embodiments of the invention the lens body comprises a polymeric or co-polymeric composition derived from a pre-polymerization mixture of monomers which comprises at least 50 weight percents acrylate monomers.

According to an aspect of some embodiments not part of the present invention there is provided a method of treating vision of a subject, comprising implanting in an eye of the subject the phakic lens device as delineated above and optionally as further detailed hereinbelow.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the described method and/or system can involve performing or completing selected tasks manually, automatically, or a combination thereof. Moreover, according to actual instrumentation and equipment of the described method and/or system, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

## BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIGs. 1A-E are schematic illustrations of a lens device, according to some embodiments of the present invention;

FIG. 2A is a schematic illustration of the lens device in embodiments of the invention in which the vaulted section comprises at least one non-elongated opening;

FIGs. 2B-D are schematic illustrations of the lens device in embodiments of the invention in which the optics-vault transition zone comprises at least one opening;

FIG. 3 is a schematic illustration of a multifocal optical section of the lens device, according to some embodiments of the present invention;

FIGs. 4A and 4B are schematic illustrations of a diffractive pattern, according to various exemplary embodiments of the present invention;

FIG. 5A is a schematic illustration of a single zone and slanted step of a conventional lens body;

FIG. 5B is a schematic illustration of a single zone and slanted step of a lens body according to various exemplary embodiments of the present invention;

FIG. 6 is a schematic illustration of geometrical definition of a zone and a step adjacent thereto, according to various exemplary embodiments of the present invention;

FIGs. 7A and 7B are schematic illustration of an outermost zone (FIG. 7A) and an innermost zone (FIG. 7B) of a prototype lens device designed according to various exemplary embodiments of the present invention;

FIG. 8A is a schematic illustration of the shape of a diffractive pattern, designed for another prototype lens device according to various exemplary embodiments of the present invention;

FIG. 8B is a schematic illustration of the diffractive pattern of FIG. 8A once formed on an aspheric side of the prototype device;

FIG. 8C is a schematic illustration of a profile view of a toric side of a prototype lens device designed according to various exemplary embodiments of the present invention; and

FIG. 9 is a schematic illustration of a lens device, once deployed according to some embodiments of the present invention, in the posterior chamber of an eye.

## DESCRIPTION OF SPECIFIC EMBODIMENTS OF THE INVENTION

The present invention, in some embodiments thereof, relates to an implantable lens device and, more particularly, but not exclusively, to a phakic lens device. Some embodiments not part of the present invention relate to a method of fabrication the phakic lens device, and some embodiments not part of the present invention relate to a method of using the phakic lens device.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Referring now to the drawings, FIGs. 1A-D illustrate a phakic lens device **100,** according to some embodiments of the present invention. FIGs. 1A and 1B are anterior (FIG. 1A) and posterior (FIG. 1B) views of lens device **100,** FIGs. 1C and 1D are left side (FIG. 1C) and right side (FIG. 1D) views of lens device **100,** and FIG. 1E is a perspective view of lens device **100.**

Lens device **100** is preferably structurally adapted to be positioned in the chamber of the eye, more preferably in the posterior chamber. In some embodiments of the present invention lens device **100** is structurally adapted to be positioned below the iris level by its entirety. Lens device **100** can be used for correction of at least one visual disorder selected from the group consisting of myopia, hyperopia, astigmatism and presbyopia.

In various exemplary embodiments of the invention lens device **100** comprises an optical section **102,** two or more haptic structures **104** and a vaulted section **108** connecting optical section **102** and haptic structures **104.** Each component of lens device **100,** including, without limitation, optical section **102,** haptic structures **104** and vaulted section **108,** can be made of any material which is sufficiently transparent to visible light and which is suitable for optics. In various exemplary embodiments of the invention at least one component of the lens device is made of biocompatible material, which can be either hydrophilic or hydrophobic. The material can be or comprise at least one substance selected from the group consisting of PMMA, HEMA, collagen and acrylic material. Representative examples of additional materials suitable for lens device **100** are provided hereinunder.

In various exemplary embodiments of the invention the lens device is foldable. The dimensions of lens device **100,** once folded, are preferably selected such as to meet the generally accepted incision sizes applied during conventional procedures for implantation phakic lenses. The dimensions of lens device, one deployed in the deployment site, are preferably selected in accordance of the sizes and distances within the anterior and/or posterior chambers of the eye of the individual mammal in which lens device **100** is to be implanted.

Haptic structures **104** are preferably generally flat. In some embodiments of the present invention haptic structures **104** are positioned at two radially opposite sides of optical part **102.** In some embodiments of the present invention, each of haptic structures **104** has at least two, or at least three outwardly protruding pads **106** for fixating haptic structures **14** in the ciliary sulcus of the eye. The advantage of having three or more pads **106** for each haptic structure is that it provides better stability of the lens device in the ciliary sulcus.

The size of lens **100,** including haptic structures **104** and pads **106,** is preferably selected such that once lens device **100** is deployed, haptic structures **104** and pads **106** engage the ciliary sulcus but do not contact the outermost circumference of the ciliary sulcus. Thus, in various exemplary embodiments of the invention the overall length of lens device **100** in its unfolded form, for a particular host eye, is longer than the largest diameter of the pupil and shorter (e.g., at least 0.25 mm shorter) than the largest diameter of the ciliary sulcus. It was found by the present Inventors that such configuration reduces the risk of pupil ovalization, interference with the accommodation in the eye and formation of cataract.

In some embodiments, lens device **100** comprises one or more marks **114** for aiding the surgeon in identifying the anterior and posterior sides of lens device **100.** In the schematic illustration of FIGs. 1A-E, marks **114** are formed in haptic structures such that when the mark is in the upper left corner of haptic structure **104,** the anterior side of the lens is facing upwards. Marks **114** can be embodied, for example, as guiding holes. However, this need not necessarily be

the case, since, for some applications, it may not be necessary for the marks to be in the form of holes. When marks **114** are in the form of guiding holes, they can have a diameter of, *e.g.,* from about 0.3mm to about 0.5mm.

Vaulted section **108** serves for maintaining lens device **100** spaced apart from the natural lens of the eye. The characteristic vaulting angle of section **108** is preferably selected to prevent excessive vaulting. It was found by the present inventors that excessive vaulting may increase the friction force between the iris and the anterior surface of lens device **100** during iris adaptation to the light conditions. Additionally, excessive vaulting may decrease the depth of anterior chamber depth and may cause endothelial cell loss. It was further found by the present inventors that excessive vaulting decreases the angle of the anterior chamber and may cause elevation of intraocular pressure, resulting in, for example, development of glaucoma. A typical vaulting depth *d* of section **108** (see FIG. 1C) is, without limitation, from about 1.1 mm to about 1.8 mm, depending on the optical power of section **102.**

Section **108** is optionally and preferably connected to haptic structures via a haptic-vault transition zone **112.** The width $w_{112}$ of zone **112** is typically from about 0.1 mm to about 0.25 mm.

In some embodiments of the present invention, vaulted section **108** comprises at least one opening **110** for allowing flow of liquid between the posterior chamber and the anterior chamber of the eye. The advantage of opening **110** is that it allows equalizing the pressures between the posterior and anterior chambers, hence reduce the risk of developing glaucoma. Openings **110** can have any shape. For example, openings **110** can have an elongated shape, *e.g.,* having a length/width aspect ratio of from about 4 to about 10. Typical dimensions of opening **110** in these embodiments include, without limitation, from about 0.3 mm to about 0.5 mm in width and from about 2 mm to about 3 mm in length. In the illustrations shown in FIGs. 1A, 1B and 1E, which is not to be considered as limiting, openings **110** have a curved shape with the convex side of the curve facing optical section **102** and the concave side of the curve facing haptic structures **104.**

FIG. 2 is a schematic illustration of lens device **100** in an embodiment in which opening **110** is not elongated. In these embodiments, the aspect ratio of opening **110** is below 2, *e.g.,* an aspect ratio of about 1. In the illustrations shown in FIG. 2, which is not to be considered as limiting, openings **110** have the shape of a circle. Typical dimensions of opening **110** in these embodiments include, without limitation, from about 0.1 mm to about 0.3 mm in diameter. When small aspect ratio openings **110** are employed, there are optionally and preferably two or more openings in section **108** between section **102** and each of haptic structures **104.**

Optical section **102** serves for correcting vision, and is therefore characterized by one or more optical power. In various exemplary embodiments of the invention section **102** is generally circular. The optical power can be refractive, diffractive or a combined refractive-diffractive optical power. Optical section **102** can be monofocal or multifocal. In some embodiments of the present invention optical section **102** comprises at least one opening **116** for allowing flow of liquid between the posterior chamber and the anterior chamber of the eye. Optionally and preferably openings **116** are arranged on the circumference of optical section **102,** as illustrated in FIGs. 1A, 1B, 1E and 2. The aspect ratio of openings **116** is preferably below 2, *e.g.,* an aspect ratio of about 1. In the illustrations shown in FIGs. 1A, 1B, 1E and 2, which is not to be considered as limiting, openings **116** have the shape of a circle. Typical dimensions of opening **116** include, without limitation, from about 0.1 mm to about 0.3 mm in diameter.

Optical section **102** is optionally and preferably connected to vaulted section **108** via a circular optics-vault transition zone **118.** Zone **118** is preferably rounded at its end **122,** as illustrated in FIGs. 1C and 1D. The advantage of having zone **118** is that it allows for smooth motion of the iris over the anterior surface of optical section **102.** In various exemplary embodiments of the invention zone **118** is devoid of optical power, other than a small refractive power (*e.g.,* less than 0.5 D or less than 0.1 D) effect by its roundness at its edge. The width $w_{118}$ of zone **118** is typically from about 0.25 mm to about 0.5 mm.

In some embodiments of the present invention zone **118** comprises at least one opening **120** for allowing flow of liquid between the posterior chamber and the anterior chamber of the eye. Optionally and preferably openings **120** are arranged symmetrically over zone **118.** For example, openings **120** can be arranged in one or more antipodal pairs with respect to the center of optical section **102,** as illustrated in FIGs. 2B-2D. In some embodiments of the present invention zone **118** comprises one antipodal pair of openings **120** (FIGs. 2C and 2D), and in some embodiments of the present invention zone **118** comprises two antipodal pairs of openings **120** (FIG. 2B). Other arrangements of openings **120** are not excluded from the scope of the present invention. The aspect ratio of openings **120** is preferably below 2, *e.g.,* an aspect ratio of about 1. In the illustrations shown in FIGs. 2B-D, which is not to be considered as limiting, openings **120** have the shape of a circle. Typical dimensions of opening **120** include, without limitation, from about 0.1 mm to about 0.3 mm in diameter.

Before providing a more detailed description of optical section **102** according to some embodiments of the present invention, a brief summary of the principles of refraction and diffraction will be provided.

When a ray of light moving in air and striking a surface of a light-transmissive substance at an angle $\alpha_1$ as measured from a normal to the surface, it is refracted into the substance at an angle which is determined by Snell's law, which is mathematically realized through the following equation:

$$n_A \sin \alpha_1 = n_S \sin \alpha_2$$

where $n_S$ is the index of refraction of the substance, $n_A$ is the index of refraction of the air, and $\alpha_2$ is the angle in which the ray is refracted into the substance. Similarly to $\alpha_1$, $\alpha_2$ is measured from a normal to the surface. A typical value of $n_A$ is about 1.

As used herein, the term "about" refers to $\pm$10 %.

Another optical phenomenon is diffraction which is the slight bending of light as it passes around the edge of an object, or at an opening thereof. The amount of bending depends on the size of the wavelength of light compared to the size of the opening or edge. If the opening is much larger than the light's wavelength, the bending will be almost unnoticeable. However, if the two are closer in size or equal, the amount of bending is considerable, and easily seen with the naked eye.

Optical effects resulting from diffraction are produced through the interaction of light waves originating from different regions of the opening causing the diffraction. Illustratively, one can view this interaction as one of two types of interferences: (i) a constructive interference when the crests of two waves combine to produce an amplified wave; and (ii) a destructive interference when a crest of one wave and a trough of another wave combine, thus canceling each other. A skilled artisan would, however, appreciate that there are many situations in which the interaction between the light waves is more complicated, *e.g.,* when the light has a plurality of wavelengths.

According to some embodiments of the present invention the refraction and diffraction phenomena are exploited for constructing a multifocal lens device. Typically, the lens device of the present embodiments has diffractive power for enabling near vision and refractive power for enabling far vision. In various exemplary embodiments of the invention the lens device also enable intermediate vision, as further detailed hereinunder.

Various embodiments of the present invention provide an optical section **102** which include both toricity and asphericity to correct or mitigate corneal astigmatism and presbyopia. The toricity and asphericity is optionally and preferably on two separate surfaces. However, embodiments in which both toricity and asphericity are present on a single surface are not excluded from the scope of the present invention. A single asphericity may be presented for all cylinder meridians or a variable asphericity may be presented for different meridians. For examples, different degrees of asphericity may be used for the two primary meridians of the astigmatism. Embodiments disclosed herein may be useful for correcting or mitigating other aberrations, such as coma, trefoil, tetrafoil, and the like. Correction of higher order aberrations are also contemplated.

Optical section **102** has an anterior surface and a posterior surface. In various exemplary embodiments of the invention one of the posterior or anterior surfaces is shaped so that Optical section **102** constitutes an aspheric lens, and in various exemplary embodiments of the invention one of the posterior or anterior surfaces is shaped so that optical section **102** constitutes a toric lens. For example, the anterior surface can be shaped so that optical section **102** constitutes an aspheric lens, and the posterior surface can be shaped so that optical section **102** constitutes a toric lens. Opposite configuration (the anterior surface constitutes a toric lens, and the posterior surface constitutes posterior an aspheric lens) are not excluded from the scope of the present invention.

FIG. 3 is a schematic illustration of a profile view of a multifocal optical section **102,** according to some embodiments of the present invention.

Optical section **102** has two opposite sides, generally shown at **12a** and **12b.** In various exemplary embodiments of the invention first side **12a** has an aspheric profile, and second side **12b** has a toric profile.

The term "aspheric profile" is well known to those skilled in the art. To the extent that any further explanation may be required, this term is employed herein to refer to a radial profile of a surface that exhibits deviations from a spherical surface. Such deviations can be characterized, for example, as smoothly varying differences between the aspherical profile and a putative spherical profile that substantially coincides with the aspherical profile at the small radial distances from the apex of the profile.

From a mathematical standpoint, an aspheric profile is a type of a conic section. A conic section can be characterized by a parameter known as a conic constant $k$, wherein $k$ > -1 corresponds to an ellipse ($k > -1$, $k \neq 0$) or a circle ($k \neq 0$), $k = -1$ corresponds to a parabola, and a $k < -1$ corresponds to a hyperbola.

For a given conic section $k$, the sag Z(s) of surface **12a** at any point s, s being the radial distance from the optical axis of optical section **102,** can be written as:

$$Z(s) = \frac{Cs^2}{1+\sqrt{1-(1+k)C^2s^2}} + A_4 s^4 + A_6 s^6 + \ldots$$

where C is the curvature (inverse of radius) of the base sphere at the optical axis, and $A_4$, $A_6$, ... are 4th, 6th, etc order

aspheric terms. The first term in the above equation describes the first order deviation of surface **12a** from a sphere, while the other terms represent higher order corrections.

In various exemplary embodiments of the invention aspheric profile **12a** is characterized by a conic constant in a range of from about -1.1 to about -3, more preferably from about -1.1 to about -1.37, inclusive. Typical values for C are, without limitation, from about 0.006 $mm^{-1}$ to about 0.1 $mm^{-1}$ (in absolute value). The values for the higher order aspheric terms can be selected such that the contribution of higher order term is less than 10% from the value of Z(s).

The term "toric profile" is also well known to those skilled in the art. To the extent that any further explanation may be required, this term is employed herein to refer to a radial profile of a surface having a first refracting power along a first meridian and a second refracting power along a second meridian, wherein the first and second meridians are perpendicular to each other and wherein the first and second refracting power differ from each other. Typically, the shape of surface **12b** is approximately that of a lateral section of a torus.

From a mathematical standpoint, a toric profile can be characterized by two conic constants $k_1$ and $k_2$, each corresponding to one of the two meridians.

For given conic sections $k_1$ and $k_2$, the sag *toric*(r, θ) of surface **12b** at any point (r, θ), r being the radial distance from the optical axis of section **102** and θ being the angle measured from the main meridian, can be written as:

$$toric(r,\theta) = \frac{(c_1 \cos^2\theta + c_2 \sin^2\theta) r^2}{1 + \sqrt{1 - (1+k_1) c_1{}^2 r^2 \cos^2\theta - (1+k_2) c_2{}^2 r^2 \sin^2\theta}}$$

where $c_1$ and $c_2$ are the curvatures along the respective meridians.

Typical values for $c_1$ and $c_2$ are, without limitation, from about 0.006 $mm^{-1}$ to about 0.1 $mm^{-1}$ (in absolute value). Typical values for $k_1$ and $k_2$ are, without limitation, from about -1.1 to about -3, or from about -1.1 to about -1.37, inclusive.

In some embodiments of the invention first side **12a** has a diffractive pattern **13** formed thereon. Optionally and preferably, diffractive pattern **13** covers at least 80% or at least 90% or at least 95% or at least 99%, *e.g.,* the entire area of surface **12a.**

Diffractive pattern **13** is preferably selected so as to provide far focus power X where X is any power from +0D to about +35D. In various exemplary embodiments of the invention diffractive pattern **13** is selected to provide add-power Y, where Y is any power from about +2D to about +4D.

The term "add-power" is well known to those skilled in the art of multifocal lenses. To the extent that any further explanation may be required, add-power refers to the amount of optical power difference between the far focus power and the near-focus power.

Preferably, X and/or Y are integers or half-integers (*e.g.,* X = 0D, 0.5D, 1D, ..., and/or Y=2D, 2.5D, 3D, ...).

A representative example of a diffractive pattern suitable for the present embodiments is provided hereinunder.

In some embodiments of the present invention the toric profile **12b** is devoid of spherical aberration, wherein a cylinder value characterizing optical section **102** is at least 1 diopter or at least 2 diopters or at least 2.5 diopters or at least 3 diopters or at least 3.5 diopters or at least 4 diopters or at least 4.5 diopters or at least 5 diopters or at least 5.5 diopters or at least 6 diopters.

A cylinder value of a lens measures the deviation from sphericity of a particular part of the lens's surface. The term "cylinder" is originated from cylindrical lenses which inherently have different foci lengths in different direction. The optical effect caused by a lens having a non-zero cylinder value is known as surface astigmatism. In optometry nomenclature, the term "astigmatism" is also used to describe a physiological defect, for example, when the cornea has an irregular curvature. For subjects with astigmatism, a certain cylinder value in the lens is desired since it corrects the eye's astigmatism.

A toric surface which is devoid of spherical aberration is also known in the literature as an "aberration neutral" surface. Use of aberration neutral surface is unlike conventional IOL devices which employ negative asphericity so as to compensate for the shape of the cornea. The present Inventors discovered that a judicious selection of the conical constants $k_1$ and $k_2$ allows the use of aberration neutral surface at the toric side, while maintaining adequate cylinder value.

FIGS. 4A and 4B illustrate a top view (FIG. 4A) and a profile view (FIG. 4B) of multifocal optical section **102,** showing diffractive pattern **13** according to various exemplary embodiments of the present invention. Diffractive pattern **13** comprises a plurality of concentric annular zones **14** separated by slanted steps **16** (shown better in FIG. 4B). The number of concentric zones is preferably at least 4, or at least 8, or at least 10, or at least 12, or at least 14, or at least 16, or at least 18, or at least 20, at least 20, or at least 22 or at least 24 or at least 26 or at least 28, or at least 29. The number of concentric zones is preferably at most 40 or at most 38 or at most 36 or at most 34 or at most 32 or at most 30. In some embodiments of the present invention section **102** comprises about 4 zones, in some embodiments of the present invention section **102** comprises about 5 zones, some embodiments of the present invention section **102** comprises about 6 zones, some embodiments of the present invention section **102** comprises about 7 zones, in some embodiments of the present invention section **102** comprises about 8 zones, in some embodiments of the present invention section **102** comprises 30 zones, and in some embodiments of the present invention section **102** comprises 40 zones.

In some embodiments of the present invention section **102** comprises 30 zones.

The device of the present embodiments differs from conventional lens devices in that substantially the entire contributions to the diffraction and refraction powers are in zones **14,** whereas the contribution of steps **16** to diffraction and refraction powers is negligible or zero, even though they are slanted with respect to the optical **18** and transverse **20** axes of optical section **102.** Thus, in various exemplary embodiments of the invention concentric zones **14** effect both diffraction and refraction of incident light, while steps **16** are substantially devoid of any diffractive or refractive power.

This advantage is illustrated in FIGS. 5A and 4B, which illustrate a single zone **24** and slanted step **26** of a conventional lens body (FIG. 5A) and a single zone **14** and slanted step **16** of optical section **102** (FIG. 5B). In FIGS. 5A-B, R1 and R2 represent refractive powers which generally equal the zeroth diffractive power, D1 represents the first order diffractive power and R0 represents a zero refractive power. In the conventional lens body, both the zones **24** and slanted steps **16** have optical power: step **16** has only a refractive power (R1) and zone **24** has both refractive (R2) and diffractive (D1) powers. In optical section **102,** on the other hand, the surface of step **16** is preferably substantially devoid of any optical (refractive or diffractive) power.

It is appreciated that whether or not there is optical power depends on the accuracy of the device which measures the optical power. As used herein, "substantially devoid of optical power" refers to zero optical power or optical power which is below 0.5 diopters, more preferably below 0.4 diopters more preferably below 0.3 diopters more preferably below 0.2 more preferably below 0.1 diopters.

Thus, every portion of optical section **102** has optical power (diffractive and/or refractive), but the contribution of this optical power generally comes from the surfaces of the zones and not the surfaces of the steps. The optical power of the surfaces of the zones is achieved by providing the zones with a finite radius of curvature relative to the transverse plane containing transverse axis **20** and/or with a secondary diffraction patterns **21** on their surface. However, the steps are preferably made planar, namely with infinite or very large radius of curvature.

The terms "refractive power" and "diffractive power" as used herein with respect to a particular optical element (either a subsection of lens optical section **102** or optical section **102** as a whole), refer to the dominant optical power of that element. Specifically, a particular optical element is said to have a refractive power if at this element the refractive power dominates the diffractive power, and particular optical element is said to have a diffractive power if at this element the diffractive power dominates the refractive power. If the refractive and diffractive powers are comparable, the element is said to have both optical powers.

In some embodiments of the present invention the diffractive power of the zones gradually decreases across each zone in the radial direction $\underline{r}$ (throughout this specification, underlined italic symbols represent vectors). This is illustrated in FIG. 5B for the case in which the diffractive power of the zone is the first order diffractive power. The advantage of gradually decreasing diffractive power is that it provides intermediate vision along with near and distance vision. Gradual decrease of the diffractive power across each zone can be achieved by changing the diffraction pattern across the zone.

The refractive power of section **102** is preferably substantially uniform across optical section **102.** For example, in some embodiments of the present invention, each zone can have the same refractive power, with deviations of less that 10% or less than 5%. Additionally, the refractive power of the zone can be substantially uniform across the refractive section of this zone. The overall effective refractive area of optical section **102** is preferably small. In some embodiments of the present invention overall effective refractive area is less than 80 % or less than 70 % or less than 60 % of the total effective area of optical section **102.**

FIG. 6 illustrates the geometrical definition of a zone **14** and a step **16** adjacent thereto. For clarity of presentation, FIG. 6 does not illustrate the entire optical section **102,** however, the center of optical section **102** is shown at **30** for reference.

Step **16** is characterized by a radius $\rho_s$ (measured, *e.g.,* from the center **30** of optical section **102** to the center of the step along radial direction r), a slope s (measured, *e.g.,* relative to the transverse plane containing transverse axis **20**), a height *H* (measured, *e.g.,* from the toric surface **12b** of optical section **102** to the tip **32** of the step), and a width $W_s$ (measured, *e.g.,* from tip **32** to the end **34** of the previous zone along radial direction $\underline{r}$).

Zone **14** is characterized by a radius $\rho_z$ (measured, *e.g.,* from the center **30** of optical section **102** to the center of the zone along radial direction $\underline{r}$), a width $W_z$ (measured, *e.g.,* from tip **32** to the beginning **38** of the next step along radial direction $\underline{r}$) and a curvature (not shown in FIG. 6). The height *H* of step **16** also characterizes zone **14.**

In some embodiments of the present invention the slope *s* and height *H* of a particular slanted step **16** are at least non-increasing functions of the radius $\rho_s$. For example, the slope and height can be decreasing functions of the radius. In other words, in this embodiment, the steps are ordered such that their slopes and heights are decreasing from center to edge. The height H and its variation over the optical section is optionally and preferably selected based on the

additional power for near focus. As a representative example for height decrease, the heights can vary from about 1.9 micron at the center of the optical section (where the radius $\rho_s$ is the smallest) to about 0.09 micron at the edge of the optical section (where $\rho_s$ is the largest). As a representative example for slope decrease, the slopes can vary from about 84° at center to about 25° at edge.

The decreasing functions of the radius of the radius can be expressed analytically. However, from a practical point of view these functions can be expressed as lookup tables. A representative example of such lookup table, for an optical section having of 30 zones and 30 steps is provided in the Example section that follows (see Table 1).

In some embodiments of the present invention steps **16** have generally the same width $W_s$, with about 10 % or less deviation. Representative examples for width W of step **16** suitable for the present embodiments includes width ranging from about 0.17 microns to about 0.2 microns, or from about 0.18 microns to about 0.19 microns, inclusive. In some embodiments of the present invention all steps have widths which are either 0.18 microns or 0.19 microns.

Preferably, the zones **14** of optical section **102** are substantially equal in area, with deviation of less than 10% or less than 5%. This embodiment is advantageous since it reduces or eliminates halos and glare.

The optical section of the present embodiments provides high level of light transmission. In various exemplary embodiments of the invention the zones and steps transmit at least 75 %, or at least 76 %, or at least 77 %, or at least 78 %, or at least 79 %, or at least 80 % of incident light.

The use of zones and steps according to various exemplary embodiments of the present invention is particularly useful from the stand point of manufacturing process. Since the steps are substantially planer, the machining of the lens is substantially simpler compared to conventional lenses.

Additional advantages of the lens device of the present embodiments over conventional vision correcting lenses include, without limitation, reduced or no aberration to the optical system, reduced or no halos and light scattering, tolerated decentration and combination of intermediate vision with far and near vision. Further, when the device is used as an intraocular lens device or contact lens device, its aspheric shape fits the surface of cornea hence making the device suitable for many patients.

The lens device of the present embodiments can be fabricated in any technique known in the art. Generally, the present embodiments form on a substance a plurality of concentric annular zones separated by slanted steps, wherein the concentric zones effect both diffraction and refraction of incident light, while the steps are substantially devoid of any diffractive or refractive power. The substance on which the zones and steps are formed can be an unprocessed or partially processed lens body, in which case the formation of zones and steps serves for forming the lens device directly. Alternatively, the substance can be a mold, in which case the formation of zones and steps serves for forming a lens mold for mass fabrication of lens devices. In these embodiments, the lens device can be cased using the lens mold, as known in the art.

The formation of zones and steps may be done by any convenient manufacturing means, including, for example, a computer-controllable manufacturing device, molding or the like.

A "computer controllable manufacturing device" refers to a device that can be controlled by a computer system and that is capable of producing directly a lens body or a mold for producing a lens device. Any known, suitable computer controllable manufacturing device can be used. Exemplary computer controllable manufacturing devices includes, but are not limited to, lathes, grinding and milling machines, molding equipment, and lasers. In various examples a Computerized Numeric Controlled (CNC) lathe machine is used, such as the lathers marketed under the trade names DAC™ Vision, Optoform and CareTec.

A Fast Tool Servo (FST) is optionally employed to form the toric profile. In these embodiments, the lathe machine can generate the diffractive pattern and the aspheric profile, and the FTS can generate the toric profile.

The present embodiments also contemplate a method of treating vision of a subject in need thereof. The method comprises implanting a multifocal lens device in an eye of the subject, thereby treating the vision of the subject. The multifocal lens device is preferably device **100** as further detailed hereinabove.

A representative implantation procedure is as follows.

Under local or global anesthesia, an incision, about 2-3 diameters in length, into the cornea **208** or limbus **210** is made to establish an access to an anterior eye chamber **200** (FIG. 9). Then, lens device **100** is introduced, for example, using a forceps or a lens delivery system (not shown), through the preliminary dilated pupil, into the posterior eye chamber **204,** below the iris **206,** *e.g.,* at 6 o'clock and then at 12 o'clock. Next, lens device **100** is adjusted for position by moving it over the outer surface of an intact natural lens **202,** while attempt is made to attain such a position at which the optical section **102** be arranged on the eye optic axis. In various exemplary embodiments of the invention the lens device **100** is positioned below the iris level by its entirety. The haptic structures **104** are introduced into the posterior chamber **206** preferably while avoiding contact between haptic structures **104** and the outermost circumference of the ciliary sulcus **212.** On completion of surgery the operative incision is stitched up.

***Compositions Suitable for the lens device***

In general, ophthalmic and ocular devices differ from spectacles (eye-glasses) and other optical devices, by being designed to be in direct contact with the living tissue of the eye or its immediate surroundings. The devices which come in such direct contact are required to be biocompatible, as discussed herein, as well as to have certain physical properties with respect to their mechanical reshapability and reformability, flexibility, water-absorption capacity, and the likes. Implantable devices differ from non-implantable devices mainly by their mode of use and administration as well as their term of use, namely the devices that require a surgical procedure in order to be put in place are typically referred to as implantable. Implantable ophthalmic and ocular devices are expected to last longer and thus the requirements for their stability and compatibility in all aspects are much higher.

In principle, the lens device can be prepared from any polymeric or co-polymeric composition which is formulated to confer the required and desired chemical and mechanical attributes, as discussed herein.

A composition used in ophthalmic applications is typically formulated to be bio-compatible, chemo-compatible and physico-compatible so as to be adequate for use within the above-described ophthalmic device, or any other ophthalmic and ocular devices.

The term "bio-compatible", as used in reference to polymeric or co-polymeric compositions as presented herein, refers to the non-toxic and benign effect that the composition has on a living tissue (e.g., an eye or a portion or a component thereof) when in contact therewith.

The term "chemo-compatible", as used in reference to polymeric or co-polymeric compositions as presented herein, refers to the long-term non-leachability of unreacted components and diluents, non-degradability and overall long-term chemical stability of the composition when in contact with a living tissue in the eye and exposed to ambient light.

The term "physico-compatible", as used in reference to polymeric or co-polymeric compositions as presented herein, refers to the optical properties of the compositions in terms of refractive index, internal reflections, light transmission and/or absorbance, and other properties relating to light-matter interaction.

The present inventors have contemplated a formation of a multifocal device as described herein from polymeric and co-polymeric compositions designed suitable for use in such a device.

The polymeric or co-polymeric compositions presented herein, are typically derived from a mixture of monomers. In polymeric compositions, the monomers in the mixture are the same whereby in co-polymeric compositions several different classes of monomers are used As in any polymer, the polymeric or co-polymeric composition is based on plurality of polymeric backbones, each of which consists of a plurality of backbone units which are covalently attached to one another, and each of the backbone units comprises a plurality of building-blocks or polymerized monomeric units. Hence, the a polymeric or co-polymeric composition as presented herein, which is the outcome of polymerizing a pre-polymerization mixture of monomers, can be characterized and defined at the chemical and physical property level, in terms of the pre-polymeric mixture, namely its constituents prior to polymerization.

The monomers which are put into the pre-polymerization mixture are still unreacted (not yet polymerized) and are regarded as the starting materials for the compositions presented herein. In the case of a co-polymeric composition as presented herein, each of the monomers, identified and classified into classes, can be characterized by chemical and physical properties exhibited by the corresponding homopolymer which is derived from each of the individual monomer in the class. Hence, the co-polymeric compositions presented herein can be characterized by the monomer class which it is derived from, as well as the relative amount-ratios between each monomer class in the pre-polymerization mixture.

As used herein, the term "monomer" is used to describe a constituent of the pre-polymerization mixture which affords, upon polymerization, the polymeric or co-polymeric compositions presented herein. It is noted herein that while some of the constituents of the pre-polymerization mixture participate in the polymerization process as reactants which form covalent bonds with other constituents in the mixture during the polymerization process, some may not react with other constituents in a covalent-bond-forming reaction, but become embedded or incorporated within the matrix of the polymeric or co-polymeric composition, as will be discussed hereinbelow.

According to some embodiments of the present invention, a pre-polymerization mixture as described herein may include any of the monomers as described herein, as a starting material, in its monomeric form, or, alternatively, as a polymeric building block, which typically has a relatively low average molecular weight of about less than 2000 Daltons (as measured via gel permeation chromatography refractive index detection). According to other embodiments of the present invention, the starting material can include dimmers of a monomer (two monomeric units or building blocks) and in some cases be a short oligomer of building blocks, including oligomers made from more than one type of monomeric unit. Short oligomers are commonly referred to in the art as "blocks".

Some of the above-described embodiments therefore relate to a lens device which includes a optical section which is formed with a plurality of concentric annular zones separated by slanted steps, wherein the concentric zones effect both diffraction and refraction of incident light, while the steps are substantially devoid of any diffractive or refractive power, as described herein, and wherein the lens device, or at least the optical section is made of a polymeric or co-polymeric composition which includes a polymeric backbone composed of a plurality of backbone units covalently linked to one another, which is derived from a pre-polymerization mixture of monomers having an improved formulations as presented hereinbelow. Optionally, according to some embodiments, the polymeric or co-polymeric composition includes at least one curcuminoid compound, as presented hereinbelow, incorporated in the composition or on the lens device, or at least the optical section as a mean to provide UV-light stabilization.

***Hydrophobic Co-polymeric Composition***

According to some embodiments of the present invention, the lens device is made of a co-polymeric composition which is uniquely designed suitable for ophthalmic devices. As discussed hereinabove, these co-polymeric compositions presented herein are formulated to be bio-compatible, chemo-compatible and physico-compatible.

Hence, according to an aspect of some embodiments of the present invention, there is provided a lens device, as described herein, wherein the lens device comprises a co-polymeric composition which is suitable for use in ophthalmic applications.

In some embodiments, the co-polymeric composition is based on five matrix-forming constituents (monomers) as well as other ingredients such as catalyst, UV-stabilizer/blocker and high energy visible blue light stabilizer. The compositions may further comprise colorant/dye additives, leachable agents (drugs) and the likes.

It is noted that the co-polymeric composition of the present embodiments is compatible for use in the eyes and is optically clear and suitable for use as material of construction of ophthalmic and ocular device. By optically clear it is meant that the composition is essentially transparent to visible light, as described and defined herein.

The co-polymeric composition presented herein typically and advantageously exhibits relatively high refractive indexes when in its final form and fully hydrated. The refractive index of the final and fully hydrated co-polymeric composition presented herein, is typically greater than about 1.5, more typically greater than about 1.51, still more typically greater than about 1.52 and even possibly greater than 1.53 or even 1.54, wherein the refractive index of the fully hydrated composition is measured at 25 °C in accordance with ASTM D 542 - 00 (2006).

Hence, according some embodiments of the present invention, the lens device presented herein comprises a co-polymeric composition, the composition being derived from a mixture of monomers that includes:

a first aromatic acrylate (aryl acrylate) monomer characterized as forming, upon polymerization thereof, a first homopolymer which exhibits a refractive index that ranges from 1.50 to 1.53;
a second aromatic acrylate (aryl acrylate) monomer characterized as forming, upon polymerization thereof, a second homopolymer, which exhibits a Tg (glass transition temperature) lower than the Tg of the homopolymer derived from the first monomer by a range of 2 to 30 degrees centigrade;
a third monomer characterized as forming, upon polymerization thereof, a third homopolymer, which exhibit a T*g* lower than 35 °C or lower than 37 °C;
a fourth monomer characterized as forming, upon polymerization thereof, a fourth homopolymer, which exhibit a capacity to absorb water to at least 20 % of its dry weight; and
a fifth monomer serving a crosslinking agent.

According to some embodiments of the invention, a concentration of the first aromatic acrylate monomer ranges from 50 % to 60 % of the total weight of the composition;
a concentration of the second aromatic acrylate monomer ranges from 15 % to 20 % of the total weight of the composition;
a concentration of the third monomer ranges from 10 % to 15 % of the total weight of the composition;
a concentration of the fourth monomer ranges from 5 % to 10 % of the total weight of the composition;
a concentration of the fifth monomer ranges from 2 % to 5 % of the total weight of the composition.

It is noted that the co-polymeric compositions presented herein, which are formed from a number of monomer classes, referred to herein as a first, second, third, fourth and fifth monomers, can include a variety of different monomers of each class, namely one or any number of monomers mentioned within the class of monomers corresponding to the first, second, third, fourth or fifth monomer.

Unless otherwise stated, the percentages (e.g., weight percentages) of the constituents of the co-polymeric composition presented herein are denoted as weight percentages of the starting material with respect to the total weight of the pre-polymerizable mixture.

According to some embodiments of the present invention, the co-polymeric composition presented herein contains less than 75 % in total of aryl acrylate monomers, keeping the major component of the co-polymeric composition based on aryl acrylate, as opposed to a mixture of aryl methacrylate and aryl acrylate known in the art, which is less suitable for ophthalmic applications in general and for the lens device presented herein.

One disadvantage of using aryl acrylate and aryl methacrylate/acrylate in more than 80 % concentration is the occurrence of internal reflections and glare which may appear in a lens device made from such co-polymeric composition.

This disadvantage is mitigated by controlling the content of the aryl (aromatic) monomer in the composition.

In general, every monomer adds from its associated property to co-polymeric composition, and when all property adds up in a prerequisite proportion, they form a co-polymer suiting the application.

The fifth monomer (class) is a crosslinking agent, as defined hereinbelow, interchangeably referred to herein as a crosslinker, which is characterized according to its capacity to alter the strength, rigidity and consistency of the co-polymeric composition presented herein. Thus, the crosslinking monomer (the fifth monomer) is a component having an effect on controlling flexibility of the obtained material for a soft embodiment of composition presented herein, giving the desired mechanical strength, improving capability of deformation recovery, and increasing co-polymerizable property with components for polymerization. Co-polymeric compositions which are not cross-linked may deteriorate rapidly as polymer chains are loosely held and increasing the possibility of getting extracted out under soxhlation extraction (gel content), resulting into loss of strength, loss of shape recovery and higher occurrence of vacuoles. The crosslinker is also the constituent that leads to an increase in the molecular weight of the composition (size of an average contiguous chain) by tethering chains to one-another. The molecular weight of the composition has a direct effect on the glistening, mechanical properties, refractive index and many other mechanical and optical properties of the composition and subsequently a device made therefrom.

The first monomer (class) is an aromatic (aryl-containing) acrylate type monomer (hence, a first aromatic monomer) which is characterized as forming, upon polymerization thereof, a first homopolymer having a refractive index between 1.50-1.53 (a criterion for selecting the first monomer). As a constituent of the co-polymeric compositions presented herein, the first monomer can include one or more monomer structures, namely different monomers wherein each satisfies at least the aforementioned criterion.

Acrylate monomers constitute a family which is a type of vinyl monomers, or esters which contain a vinyl group, namely two carbon atoms double-bonded to each other, directly attached to the carbonyl carbon of a carboxyl group, as illustrated in Scheme 1 below.

*Scheme 1*

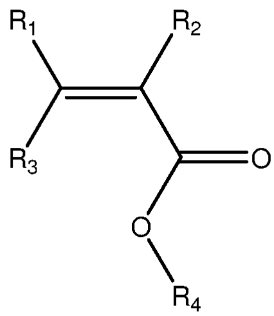

The term "methacrylate" refers to an acrylate monomer having a methyl group at position $R_2$ in Scheme 1 above.

The phrase "aromatic acrylate", as used herein, refers to an acrylate ester having an aromatic substituent attached to the carbonyl, denoted $R_4$ in Scheme 1 above.

Accordingly, the phrase "aromatic methacrylate" refers to a monomer as illustrated in Scheme 1 above, wherein $R_2$ is a methyl group and $R_4$ is an aryl or a heteroaryl group.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted.

Without being bound by a particular theory, the rationale behind using aryl ether acrylate monomers is their relatively flexible result-polymer compared to straight chain aryl alkyl methacrylate which adds to greater deforming ability into lens matrix without significantly compromising on refractive index and hydrophobicity.

Exemplary monomers that are suitable for use as a first monomer according to embodiments of the invention include 2-phenoxyethyl acrylate, 2-phenoxy ethyl methacrylate, 2-benzyloxy ethyl acrylate, 2-benzyloxy ethyl methacrylate and combinations thereof.

Other non-limiting examples of the first aromatic acrylate monomer according to some embodiments of the present invention, include 2-ethylphenoxy methacrylate; 2-ethylphenoxy acrylate; 2-ethylthiophenyl methacrylate; 2-ethylthiophenyl acrylate; 2-ethylaminophenyl methacrylate; 2-ethylaminophenyl acrylate; phenyl methacrylate; phenyl acrylate; benzyl methacrylate; benzyl acrylate; 2-phenylethyl methacrylate; 2-phenylethyl acrylate; 3-phenylpropyl methacrylate; 3-phenylpropyl acrylate; 4-phenylbutyl methacrylate; 4-phenylbutyl acrylate; 4-methylphenyl methacrylate; 4-methylphenyl acrylate; 4-methylbenzyl methacrylate; 4-methylbenzyl acrylate; 2-2-methylphenylethyl methacrylate; 2-2-methylphenylethyl acrylate; 2-3-methylphenylethyl methacrylate; 2-3-methylphenylethyl acrylate; 24-methylphenylethyl methacrylate; 2-4-methylphenylethyl acrylate; 2-(4-propylphenyl)ethyl methacrylate; 2-(4-propylphenyl)ethyl acrylate; 2-(4-(1-methylethyl)phenyl)ethyl methacrylate; 2-(4-(1-methylethyl)phenyl)ethyl acrylate; 2-(4-methoxyphenyl)ethyl methacrylate; 2-(4-methoxyphenyl)ethyl acrylate; 2-(4-cyclohexylphenyl)ethyl methacrylate; 2-(4-cyclohexylphenyl)ethyl acrylate; 2-(2-chlorophenyl)ethyl methacrylate; 2-(2-chlorophenyl)ethyl acrylate; 2-(3-chlorophenyl)ethyl methacrylate; 2-(3-chlorophenyl)ethyl acrylate; 2-(4-chlorophenyl)ethyl methacrylate; 2-(4-chlorophenyl)ethyl acrylate; 2-(4-bromophenyl)ethyl methacrylate; 2-(4-bromophenyl)ethyl acrylate; 2-(3-phenylphenyl)ethyl methacrylate; 2-(3-phenylphenyl)ethyl acrylate; 2-(4-phenylphenyl)ethyl methacrylate; 2-(4-phenylphenyl)ethyl acrylate; 2-(4-benzylphenyl)ethyl methacrylate; and 2-(4-benzylphenyl)ethyl acrylate, and the like.

Additional examples for suitable first monomers include naphthyl acrylates, dicyclopentyloxy acrylates, dicyclopentyl acrylates, nonylphenoxy polyethyleneglycol 200 acrylates, nonylphenoxy polyethyleneglycol 400 acrylates, alkoxylated phenol acrylates, 2-methacryloyloxyethyl 2-hydroxy propyl phthalates, 2-acryloxy ethyl-2-hydroxy ethyl phthalates, 2-hydroxy-3-phenoxy propyl acrylates, neopentyl glycol benzoate acrylates and the likes.

According to some embodiments of the present invention, the concentration of the first monomer ranges from 52 % to 59 % of the total weight of the composition.

The second monomer (class) is another aromatic acrylate monomer characterized as forming, upon polymerization thereof, a second homopolymer having a Tg lower than the T*g* of the first homopolymer, which is derived from the first monomer, by 2 to 30 degrees centigrade (°C).

Non-crystalline polymeric solids are referred to as amorphous materials (atoms or molecules are not arranged in a lattice that repeats periodically in space). For all amorphous solids, whether glasses, organic polymers, and even metals (although having a lattice), T*g* is the critical temperature that separates their glassy and rubbery behaviors. A glass is defined as a material that has no long-range atomic or molecular order and is below the temperature at which a rearrangement of its atoms or molecules can occur. On the other hand, a rubber is a non-crystalline solid whose atoms or molecules can undergo rearrangement. If a material is at a temperature below its T*g*, large-scale molecular motion is not possible because the material is essentially frozen. If it is at a temperature above its Tg, molecular motion on the scale of its repeat unit (such as 50-mer in a polymer) takes place, allowing it to be "soft" or "rubbery". It is noted that the term "T*g*" is applies herein to non-crystalline solids, which are mostly either "glasses" or "rubbers".

Hence, the phrases "glass transition temperature" or "rubber-glass transition temperature", as used herein in the context of polymers, refers to the temperature at which the Gibbs free energy is such that the activation energy for the cooperative movement of about 50 elements (50-mer) of the polymer is exceeded compared to a reference point, meaning that molecular chains are able to slide past each other when a force is applied. A glass transition temperature of a non-crystalline material, such as a polymer, is the critical temperature at which the material changes its behavior from being "glassy" to being "rubbery", while lowering the temperature across the T*g* affords vitrification. "Glassy" in this context means hard and brittle (and therefore relatively easy to break), while "rubbery" means elastic and flexible and can absorb kinetic energy without shuttering.

According to some embodiments of the present invention, the chemical structure of the second monomer can follow the same chemical rational characterizing the first monomer, with the difference that the second monomer is selected according to the Tg characterizing the second homopolymer being lower than the Tg of the first homopolymer by 2-30 °C.

The choice of a second aromatic monomer according to embodiments of the invention include, depends on the choice of the first monomer as difference in relative Tg is the criterion for selecting the second monomer. Therefore, there is an overlap in the range of options for the first and the second monomers.

Exemplary monomers that are suitable for use as a second monomer according to embodiments of the invention include, but are not limited to 2-phenylethyl acrylate, benzyl acrylate, 2-chlorophenyl acrylate, 4-methyl benzyl acrylate, 2,4,6-tribromophenyl acrylate, pentabromophenyl acrylate and any combinations thereof.

According to some embodiments of the present invention, the concentration of the second monomer ranges from 15 % to 19 % of the total weight of the composition.

It is noted herein that some ophthalmic and ocular devices, and particularly implantable devices, are required to avoid posterior capsular opacification (PCO) after cataract replacement surgery. This adverse effect relates also to the tackiness of the composition. To control tackiness, suitable monomers are selected for the co-polymeric composition, while not compromising the refractive index of the resulting composition. Hence, the co-polymeric composition presented herein exhibits tackiness to some degree so as to avoid PCO, and therefore tackiness should be high enough to reduce PCO, and low enough so as not to hinder handling.

Thus, the first monomer is meant to be restricted to the above-mentioned range, since the first monomer is

characterized by a relatively higher Tg which makes the resulting polymer less tacky than the polymer resulting from the second monomer. These formulation restrictions confer relatively low tackiness without compromising the target refractive index. At the same time, the reason for keeping a high Tg monomer (higher than a low Tg monomer), is to confer mechanical strength to the lens device, which increases due to higher crystallinity imparted to the resulting polymer.

It is further noted herein that according to some embodiments of the present invention, a relatively high Tg aryl monomer is used as a major constituent, while the prior art teaches the use of lower Tg aryl monomer as a major part of the composition. For example, U.S. Patent No. 5,290,892 teaches the use of 2-phenyl ethyl acrylate in higher amount compared to 2-phenyl ethyl methacrylate, while methacrylate compounds have higher Tg compared to corresponding acrylate compound. Together it forms at least 80 % of the composition taught in U.S. Patent No. 5,290,892.

The third monomer (class) is characterized as forming, upon polymerization thereof, a third homopolymer having a T*g* lower than 37 °C. Thus, the third monomer is the component which has a notable effect on the flexibility of the obtained composition of the lens device presented herein, conferring softness and improving its capability to recover from deformation. The capacity to recover quickly from deformation (reformability) is required by an ophthalmic device when applied to the eye after folding and while following the shape shifts of the eye.

Exemplary monomers that are suitable for use as a third monomer according to embodiments of the invention include, but are not limited to, cellosolve methacrylate, methoxy ethyl acrylate, polyethylene glycol monomethacrylate, 1-dihydroxyperflurobutyl methacrylate, 2,5-dibromopropyl methacrylate, hexyl methacrylate, glycerol monomethacrylate, trifluroethyl methacrylate, butyl methacrylate, n-ocyl/isooctyl methacrylate, n-decyl/isodecyl methacrylate, ethyl methacrylate, ethylene triglycol methacrylate, butyl diglycol methacrylate, methoxy polyethylene glycol 350 methhacrylate, methoxy polyethylene glycol 500 methhacrylate, methoxy polyethylene glycol 1000 methhacrylate, methoxy polyethylene glycol 2000 methhacrylate. methoxy polyethylene glycol 5000 methhacrylate, polypropylene glycon methacrylate, ethoxytriglycol methacrylate, 2-ethoxyethoxy ethyl methacrylate, methoxy triethyleglycol methacrylate, phenoxy polyethylene glycol monomethacrylate and any combinations thereof.

According to some embodiments of the present invention, the concentration of the third monomer ranges from 11 % to 15 % of the total weight of the composition.

It is noted herein that use of aryl ether acrylate and aryl alkyl acrylate, such as 2-phenoxy ethyl acrylate and 2-phenyl ethyl acrylate, gives superior results over the use of methacrylate and acrylate of same pendant group as described in the art.

Methacrylate groups are known to increase the strength and rigidity of the resulting composition, since methacrylate compounds exhibit side chain crystallization thereby increasing strength and rigidity. According to some embodiments of the present invention, it is suggested herein to introduce high Tg aryl ether acrylate monomer which also imparts flexibility to the co-polymeric composition, enabling 20D IOL delivery even through a sub 2 mm incision in a wound assisted surgical technique. An additional advantage of using aryl ether acrylate is its relatively less tacky nature compared to aryl alkyl methacrylate.

By using lesser amount of aryl acrylate monomer, polymer of relatively low refractive index is prepared to avoid glare/internal reflection problem at the same time, refractive index is maintained to a level that it would enable the lens to go through sub 2 mm incision.

For increasing strength and reformability, monomers like methoxy ethyl methcaylate are used. Its Tg is lower than 37 °C and it forms relatively hydrophobic polymer than conventional 2-ethoxy ethyl methacrylate. In general, in the pendant ether group, odd number of methylene ($CH_2$) gives optimal results. Examples include methoxy ethyl acrylate, propoxy ethyl acrylate, pentoxy ethyl acrylate and the likes.

The fourth monomer (class) is a hydrophilic monomer which is characterized as forming, upon polymerization thereof, a fourth homopolymer exhibiting a capacity to absorb water to at least 20 % of its dry weight.

Water content of the homopolymer made from hydrophilic monomer should absorb enough so as to conform to the requirement of ophthalmic devices such as contact lenses and IOLs. After extraction of the leachables from the lens device, a process that is discussed hereinbelow, vacuoles may form in the product. Hence, all hydrophobic polymeric compositions exhibit vacuoles and some water absorption. When ophthalmic device comes in a contact with water or another aqueous medium, these media would tend to concentrate at the vacuoles.

Prime purpose of using a hydrophilic monomer is to uniformly disperse the water in the matrix. After putting the ophthalmic device in physiological medium like normal saline or highly pure water, these vacuoles give rise to white spots. To overcome this problem, hydrophilic monomer would disperse water uniformly rather than allowing water to concentrate in voids and vacuoles. This uniform dispersion gives rise to clear and spotless lenses. It also helps to increase the strength of the matrix and to control its tackiness.

Some commonly available hydrophilic monomer absorbs more than 20 % of the total weight of their corresponding homopolymer. If the required water content in the lens device should be kept bellow 20 %, other monomers can be selected so as to counter-effect the absorption of water.

The forth monomer is also effective to reduce tackiness of the co-polymeric composition presented here, as well as to improve its mechanical properties, as well as to uniformly disperse water molecules throughout the matrix at

a wide temperature change.

As used herein, the phrase "hydrophilic monomer" refers to compounds which produce hydrogel-forming homopolymers, namely homopolymers which become associated with substantial amounts of water (for example, at least 20 % based on the weight of the dry homopolymer), and which physically swell as a result of such association.

Exemplary fourth monomers include, without limitation, alkoxy alkyl (meth)acrylate; N-vinyl pyrrolidone; hydroxyalkyl acrylates and hydroxyalkyl methacrylates, such as 2-hydroxyethyl acrylate, 2-hydroxyethyl methacrylate, 3-hydroxypropyl acrylate, 3-hydroxypropyl methacrylate, 4-hydroxybutyl acrylate, 4-hydroxybutyl methacrylate, 2,3-dihydroxypropyl acrylate, 2,3-dihydroxypropyl methacrylate and the like; acrylamide; N-alkyl acrylamides such as N-methyl acrylamide, N-ethyl acrylamide, N-propyl acrylamide, N-butyl acrylamide and the like; acrylic acid; methacrylic acid; and the like and mixtures thereof.

Additional exemplary monomers that are suitable for use as a fourth monomer according to embodiments of the invention include, but are not limited to, hydroxyl ethyl methacrylate, glycerol monomethacrylate, ethylene triglycol methacrylate, butyl diglycol methacrylate, methoxy polyethylene glycol 350 methhacrylate, methoxy polyethylene glycol 500 methhacrylate, methoxy polyethylene glycol 1000 methhacrylate, methoxy polyethylene glycol 2000 methhacrylate, methoxy polyethylene glycol 5000 methhacrylate, polypropylene glycon methacrylate, ethoxytriglycol methacrylate, methoxy triethyleglycol methacrylate, phenoxy polyethylene glycol monomethacrylate and any combinations thereof.

According to some embodiments of the present invention, the concentration of the fourth monomer ranges from 7 % to 9 % of the total weight of the composition.

As used herein, the phrase "cross-linking monomer" refers to a substance that promotes or regulates intermolecular covalent, ionic, hydrophobic or other form of bonding between polymer chains, linking them together to create a network of chains which result in a more rigid structure. Crosslinking monomers, according to some embodiments of the present invention, contain at least two reactive groups that are reactive towards a variety of groups, including double bonds, sulfhydryls and amines, and create chemical bonds between two or more polymer molecules. Crosslinking monomers include homo-bifunctional crosslinking monomers that have two identical reactive end groups, and hetero-bifunctional crosslinking monomers which have two different reactive end groups. These two classes of crosslinking monomers differ primarily in the chemical reaction which is used to effect the crosslinking step, wherein homo-bifunctional crosslinking monomers will require a one step reaction, and hetero-bifunctional crosslinking monomers will require two steps to effect the same. While homo-bifunctional crosslinking monomers have the tendency to result in self-conjugation, polymerization, and intracellular cross-linking, hetero-bifunctional agents allow more controlled two step reactions, which minimize undesirable intramolecular cross reaction and polymerization. Crosslinking monomers are further characterized by different spacer arm lengths. A crosslinking monomer with a longer spacer arm may be used where two target groups are further apart and when more flexibility is desired.

Exemplary crosslinking monomers include, without limitation, butanediol di(meth)acrylate, ethylene glycol di(meth)acrylate, diethylene glycol di(meth)acrylate, triethylene glycol di(meth)acrylate, propylene glycol di(meth)acrylate, dipropylene glycol di(meth)acrylate, diallyl fumarate, allyl (meth)acrylate, vinyl (meth)acrylate, trimethylolpropane tri(meth)acrylate, methacryloyloxyethyl (meth)acrylate, divinylbenzene, diallyl phthalate, diallyl adipate, triallyl diisocyanate, $\alpha$-methylene-N-vinylpyrrolidone, 4-vinylbenzyl(meth)acrylate, 3-vinylbenzyl (meth)acrylate, 2,2-bis((meth)acryloyloxyphenyl)hexafluoropropane, 2,2-bis((meth)acryloyloxyphenyl)propane, 1,4-bis(2-(meth) acryloyloxyhexafluoroisopropyl)benzene, 1,3-bis(2-(meth)acryloyloxyhexafluoroisopropyl)benzene, 1,2-bis(2-(meth) acryloyloxyhexafluoroisopropyl)benzene, 1,4-bis(2-(meth) acryloyloxyisopropyl)benzene, 1,3-bis(2-(meth) acryloyloxyisopropyl) benzene, 1,2-bis(2-(meth) acryloyloxyisopropyl)benzene, and the like. These crosslinking monomers can be used solely or in a combination use of two or more thereof. Among those, ethylene glycol dimethacrylate and butanediol diacrylate are widely use effect controllable flexibility, desired mechanical strength, improved capability of deformation recovery, and increased co-polymerizable property.

Additional exemplary monomers that are suitable for use as a fifth monomer according to embodiments of the invention include, but are not limited to ethylene glycol dimethacrylate, 1,4-butane diol diacrylate, glycerol dimethacrylate, allyl methacrylate, 1,6 heaxane diol diacrylate, 1,4-butanediol dimethacrylate, 1,6-hexane diol dimethacrylate and any combinations thereof.

According to some embodiments of the present invention, the concentration of the fifth monomer ranges from 2 % to 3.5 % of the total weight of the composition.

The amount of the fifth monomer (crosslinker) depends on selection of the prior monomers. It is added to have an optimal shape recovery, to lessen the extractables and reduce glistening. An excess amount may increase glistening of the lens device after introduction into physiological medium. Lesser amount may result into loss of strength, higher extractables (more vacuoles) and slower shape recovery.

In addition, the co-polymeric compositions presented herein can include a variety of additional or alternative ingredients, feature additives and the likes. Examples include, without limitation, UV blockers, dyes, light-stabilizers, coating materials, pharmaceuticals (therapeutic agents), cell receptor functional groups, protein groups, viscosity agents (e.g., thickeners or thinners), diluents, combinations thereof or the like.

As discussed hereinabove, each and every constituent in the co-polymeric compositions presented herein, as well as their relative proportion in the making thereof, contribute to the wide range of requirements and necessary characteristics of lens device made from these compositions.

For example, according to some embodiments of the present invention, the lens device made from the compositions presented herein exhibit visible light transmission of at least 97 % of incident visible light, as determined according to ASTM D 1003 standard and/or ISO 11979-2:2000 standard.

In addition, or alternatively, the lens device made from the compositions presented herein exhibit a refractive index of at least 1.53, as determined according to the ASTM D 542-00(2006) standard.

In addition, or alternatively, according to some embodiments of the present invention, the compositions used to manufacture the lens device presented herein exhibit a loop pull force mechanical strength of at least 60 grams, at least 50 grams or at least 40 grams, as determined according to the ISO 11979-3:2006 standard.

In addition, or alternatively, the compositions used to manufacture the lens device presented herein are characterized by a glass transition temperature not higher than 5 °C, not higher than 10 °C or not higher than 15 °C, as determined according to the ASTM D3418-03:2000 standard.

In addition, or alternatively, Shore A hardness exhibited by lens device prepared from the compositions according to some embodiments of the present invention, ranges from 77 to 80, as determined according to the ASTM D2240:2000 standard.

In addition, or alternatively, according to some embodiments of the present invention, the co-polymeric compositions presented herein exhibit an unfolding time of less than 6 seconds for full recovery of original shape injected through a sub 2 mm cartridge at room temperature. Such requirement is important when the device is placed in position by inserting it under leaving tissue with a narrow gauge cartridge (sub 2 mm).

When using the co-polymeric compositions according to some embodiments of the present invention, the resulting lens device is characterized by having essentially no internal reflections, and further have essentially no vacuoles and/or perceivable glistening as determined by visual inspection at a magnification of 50X.

One of the requirements of the lens device presented herein, involving leachable content, is clearly met by the co-polymeric compositions presented herein, which have a leachable content of less than 0.6 %, as determined according to the ISO 11979-5:2006 and/or the ISO 11979-5:2006 standards.

According to some embodiments of the present invention, the lens device made from the compositions presented herein are essentially tack-free, as determined according to the ASTM D 3654 standard.

The lens device made from the co-polymeric compositions presented herein can also be referred to as a hydrophobic diffractive ophthalmic device and can be, for example, a hydrophobic diffractive IOL.

According to some embodiments of the present invention, the co-polymeric composition for making the device or body lens of the device described herein further includes a radiation-resistant compound, which is typically referred to as a UV-blocker agent or additive, UV-light stabilizer and/or UV-absorbent agent. The terms "UV-blocker" and "UV-stabilizer", and grammatical diversions and inflections thereof, are used herein interchangeably, since stabilizing a polymeric composition against the degradation caused by UV-light stems also from the capacity to block UV-light by the composition.

The role of protection from UV damages, both to the eye (protect light sensitive retina) and the co-polymeric composition itself, can be taken by one type of UV-blocker or a combination of several different compounds, some embedded and some copolymerized with the composition. The UV-blocker can thus be a polymerizable constituent, which presents advantages in terms of leachability of the agent, and it can be an embedded constituent, namely incorporated consistently in the matrix of the polymeric composition so as not to leach out.

It is noted herein that UV-protection is particularly desirable for ophthalmic devices such as the lens device presented herein, which may be located within the eye for extended periods of time (e.g., greater than 6 months, a year, several years or more) as opposed to, for example, disposable contact lenses. As such, it is highly desirable for these types of devices to exhibit longer term resistance to degradation caused by radiation exposure.

An ultra-violet absorbing material (UV-blocker additive) can be any natural or synthetic compound which absorbs ultraviolet light, i.e., light having a wavelength shorter than about 400 nm, but does not absorb any substantial amount of visible light. A natural UV-blocker can be a curcuminoid compound, as defined and discussed hereinbelow. The ultraviolet absorbing compound is incorporated into the monomer mixture and is embedded or entrapped in the polymer matrix when the monomer mixture is polymerized. According to some embodiments of the present invention, the UV-blocker provide a transmission cut-off above a wavelength of 385 and typically provide cut-off in the short wavelength visible (410-430 nm) region of the electromagnetic spectrum. Such chromophores can then provide desired protection to the human eye and/or the device material from UV radiation (<400 nm). Suitable UV-blockers can also be referred to as UV/short wavelength visible light absorbers, dye or chromophores.

Unless otherwise specified, "cut-off' means the wavelength at which light transmission does not exceed 1 %. "1 % cut-off' means the wavelength at which light transmission does not exceed 1 %. "10 % cut-off' means the wavelength at which light transmission does not exceed 10 %.

Typical ultraviolet absorbing compounds, based on synthetic chromophores, include substituted benzophenones, such as 2-hydroxybenzophenone, and 2-(2-hydroxyphenyl)benzotriazoles. According to some embodiments of the present invention, the ultraviolet absorbing compound may be co-polymerizable with the monomers and is thereby firmly embedded in the polymer matrix. In this way possible leaching of the ultraviolet absorbing compound out of the lens and into the interior of the eye is minimized. Suitable co-polymerizable ultraviolet absorbing compounds include substituted 2-hydroxybenzophenones as disclosed in, for example, U.S. Patent No. 4,304,895 (incorporated by reference as fully set forth herein) and 2-hydroxy-5-acryloxyphenyl-2H-benzotriazoles disclosed in U.S. Patent No. 4,528,311 (incorporated by reference as fully set forth herein). Alternatively, the ultraviolet absorbing compound is 2-(3'-methallyl-2'-hydroxy-5'methyl phenyl) benzotriazole.

Other synthetic ultraviolet absorbing compounds include phenol-2-(5-chloro-2H-benozotriazol-2-yl)-6-(1,1-)dimethyl-4-methyl (Tinuvin® 326), 4-benzoyl-3-hydroxyphenyl-2-methacrylate, 2-[4-(2h-1,2,3-benzotriazol2-yl)-3-hydroxyphenoxy]ethyl-2-methacrylate and combination thereof.

Optic devices based on co-polymeric compositions may also comprise a polymerizable or embedded yellow dye that attenuates medium- to long-wavelength (430-500 nm) blue light. Such dyes and other useful chromophores are described in U.S. Patent No. 7,691,918, which is fully incorporated herein for all purposes.

Yellow dye gives a yellowish tint to lens; natural lens tends to get yellow as age of patient progresses. Yellow tinted artificial lens gives the elderly patient the appearance of a natural lens. The yellow dye also provides protection from visible blue light which may lead to age related macular degeneration.

Presently known UV-blocking additives and synthetic chromophores, used in polymeric compositions for ophthalmic and ocular devices, may suffer from one or more drawbacks, including biocompatibility, physical, mechanical and chemical stability, and manufacturing factors (e.g., cost and complex syntheses).

***Polymeric and co-polymeric compositions containing Curcuminoid as a Natural UV-Blocker:***

According some embodiments of the present invention, lens device presented herein, which comprises a co-polymeric composition (also referred to herein as a co-polymer or a hydrophobic co-polymeric composition) described herein, further includes a natural UV-blocker in the form of a curcuminoid compound incorporated in or on the co-polymeric composition and/or the lens device.

According to some embodiments of the present invention, the pre-polymerization mixture of monomers includes at least 50 percents acrylate monomers.

It is noted herein that the benefits of incorporating a curcuminoid compound in ophthalmic and ocular devices applies to any polymeric or co-polymeric composition or a lens section of a device comprising the same. Thus, an ophthalmic or ocular device, according to some embodiments of the present invention, can be made from any suitable polymeric or co-polymeric composition as known in the art, and the curcuminoid compound can be added to the pre-polymerized mixture of the composition before curing, or applied thereon after curing, as described herein.

According to another aspect of the present invention, there is provided a lens device which includes a optical section which is formed with a plurality of concentric annular zones separated by slanted steps, wherein the concentric zones effect both diffraction and refraction of incident light, while the steps are substantially devoid of any diffractive or refractive power, wherein the lens device is made of a polymeric or co-polymeric composition which includes at least one curcuminoid compound, as presented hereinabove, incorporated in the composition or on the lens device as a mean to provide UV-light stabilization.

Lens device can be made from any suitable polymeric or co-polymeric composition as known in the art, and the curcuminoid compound can be added to the pre-polymerized mixture of the composition before curing, or applied thereon after curing, as described herein. The curcuminoid compound is therefore incorporated into the composition as described hereinabove.

In some embodiments, a polymeric or co-polymeric composition which includes a curcuminoid compound is derived from a pre-polymerization mixture of monomers that includes at least 50 weight percents acrylate monomers.

Exemplary acrylate monomers suitable for use in the context of these embodiments include, but are not limited to, an acrylate, a methacrylate, an aryl acrylate and an aryl methacrylate.

As demonstrated in the Examples section that follows, one exemplary co-polymer which includes a curcuminoid compound can be formed from one or more monomers such as, but not limited to, 2-phenoxy ethyl methacrylate (POEMA), cyclohexyl acrylate (CHMA) and 1,4-butane diol diacrylate (BDDA). An exemplary composition, according to some embodiments of the present invention, is formed, for example, from a pre-polymerization mixture containing 50-70 % 2-phenoxy ethyl methacrylate (POEMA), 20-50 % cyclohexyl acrylate (CHMA) and 1-5 % 1,4-butane diol diacrylate (BDDA), and 0.001-0.5 % curcuminoid compound, each measured by dry weight percentages of the total dry weight of the pre-polymerization mixture. Other constituents may also be included in minor amounts, such as a catalyst (a polymerization initiator agent), cross-linking monomers, colorant/dye additives and the likes.

Optionally, according to embodiments of this aspect, the co-polymeric composition includes a polymeric backbone composed of a plurality of backbone units covalently linked to one another, which is derived from a pre-polymerization mixture of monomers having a unique formulations as presented hereinabove.

Hence, according to another aspect of some embodiments of the present invention, there is provided a lens device which includes an optical section which is formed with a plurality of concentric annular zones separated by slanted steps, wherein the concentric zones effect both diffraction and refraction of incident light, while the steps are substantially devoid of any diffractive or refractive power, wherein the optical section is made of a hydrophobic co-polymeric composition, as described herein.

Combining the beneficial features imparted by the curcuminoid compound as described herein with the beneficial features of the co-polymeric composition described herein result in a vacuole free, glistening free, internal reflection free and tack free co-polymeric composition, which is protected from the damaging effects of UV light and meet the requirement of tensile strength, deformation recovery ability and mechanical, optical, biological and toxicological requirements.

As can be seen in the Examples section that follows, the present inventors have successfully incorporated curcumin in an exemplary co-polymeric composition which is suitable for use in ophthalmic and ocular devices such as the lens device discussed hereinabove, and were able to obtain a desirable level of transparency towards visible light and at the same time opacity with respect to ultraviolet light, using a relatively low concentration of curcumin.

Thus, it has been demonstrated that naturally occurring (natural) compounds such as curcuminoids, which are generally recognized as safe for human consumption and somatic use, can be used with certain polymers or co-polymers, such as substantially acrylate-based polymers, to form lasting compositions with suitable UV/blue light blocking and UV-light stabilization properties. It has been recognized that curcuminoids used in ophthalmic and ocular devices as blue/UV light blockers would overcome the limitations and possible impairments associated by the use of synthetic compounds such as diphenyl-azo-based, benzotriazole-based and benzophenone-based UV-blockers and the like.

It has been further demonstrated by some of the present inventors that curcuminoids can be used effectively even at low concentration in the polymeric composition, relative to the concentration required for benzophenone-based and benzotriazole-based UV-blockers, in order to achieve comparable effective UV-blocking. A low concentration of the UV-blockers not only affects the cost of the resulting product but also affects the visual clarity of the product and its final dimensions. Furthermore, the proven UV-blocking effectiveness of curcuminoids would make it highly suitable for in the lens device presented herein.

Thus, relatively low concentrations of the curcuminoids are sufficient to exhibit the desired activity. Hence, according to some embodiments of the present invention, the concentration of the curcuminoid compound in the composition ranges from 0.0002 weight percentage to 1 weight percentage or from 0.001 weight percentage to 0.5 weight percentage of the total weight of the composition. Any value lower than 1 weight percent is contemplated, hence any value lower than 0.9, 0.8, 0.7, 0.6, 0.5, 0.4, 0.3, 0.2, 0.1, 0.05, 0.001 and any value lower than 0,0005 weight percent is contemplated as well.

It has been further demonstrated that curcuminoids, unlike many known synthetic phenol-containing UV-blockers, do not inhibit or retard the rate and/or degree of polymerization, when added to the pre-polymerization mixture.

The use of compounds of the curcuminoid family allows selecting a compound with certain optical characteristics, such as, for example, a particular light-absorption range and a particular color or lack thereof.

Without being bound by any particular theory, it is suggested that the transparency of the co-polymeric composition is maintained upon incorporating curcumin therein due to the relatively low concentration of the embedded curcumin. Such low concentration is sufficient due to the effective UV-absorption characteristics of curcuminoids and the surprising finding that curcuminoids do not leach out of polymers or co-polymers derived from pre-polymerization mixtures having at least 50 percents acrylic monomers.

As further demonstrated in the Examples section that follows, curcumin was found to be incorporated firmly and consistently in an exemplary polymeric composition, as confirmed by the stability of its concentration in the tested composition before and after the tested composition was subjected to extended cycles of extraction in organic solvents which are known to dissolve curcumin.

The term "incorporated", as used herein, refers to the physical state of one substance in a composition containing other substances. In the context of some embodiments of the present invention, an incorporated curcuminoid compound is incorporated within the co-polymer composition described herein such that the curcuminoid compound is at least partially surrounded by the co-polymeric composition and entrapped thereby.

In some embodiments, the incorporated curcuminoid compound is distributed within the polymeric or co-polymeric composition in a uniform and sustainable form, and is enclosed in the surrounding mass.

According to some embodiments of the present invention, the curcuminoid compound incorporated within the polymeric or co-polymeric composition is not covalently attached to one or more constituents of the polymeric or co-polymeric composition. In some embodiments, the curcuminoid compound interacts with the polymeric or co-polymeric composition via physical interactions, such as, for example, entanglement, absorption, adsorption and/or entrapment, and not via chemical interactions such as covalent, ionic, or hydrogen bonds.

Curcuminoids constitute a versatile group of chromophore-containing substances, which can be selected by

their light absorption properties to suit a particular application.

In general, curcuminoids are polyphenols characterized by a pronounced yellow color, and most of the naturally occurring curcuminoids, including curcumin itself, have been recognized as generally safe for human consumption and suitable for pharmaceutical purposes.

The term "curcuminoid", as used herein, is used to collectively describe curcumin, as well as derivatized curcumin compounds. Derivatized curcumin compounds have a curcumin backbone structure, and optionally have one or more different chemical groups (substituents) attached at various positions of the curcumin backbone structure. A derivatized curcumin compound may differ from curcumin by chemical and physical characteristics, such as solubility, reactivity, light interaction and the likes, as a result of its substituents.

Curcuminoid compounds according to some embodiments of the present invention, can be collectively represented by General Formula I:

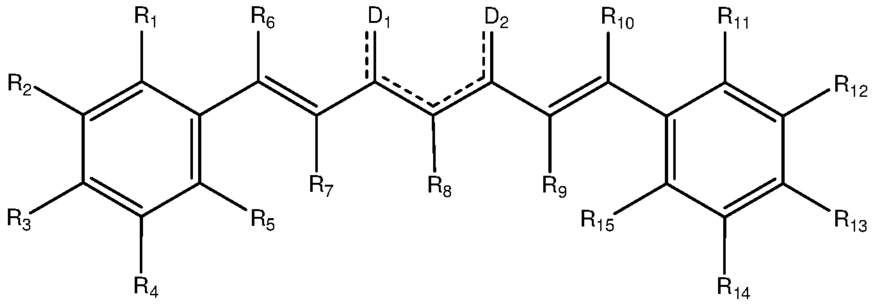

***Formula I***

According to some embodiments of the present invention, each of $D_1$ and $D_2$ is individually selected from the group consisting of O, N, S or C(aryl), whereas $D_1$ and $D_2$ may be connected directly or via a connecting atom to form a conjugated ring (aryl, heteroaryl etc.); and wherein each of $R_1$-$R_{15}$ is individually selected from the group consisting of alkyl, alkoxy and hydroxy.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted.

The curcuminoid compound utilized in embodiments of the present invention can be a naturally-occurring curcuminoid or a synthetically prepared curcuminoid, with naturally-occurring curcuminoids being more desirable.

Exemplary curcuminoid compounds that are suitable for use in the context of some embodiments of the present invention include, but are not limited to, curcumin (illustrated in the scheme below), bisdemethoxycurcumin, monodemethoxycurcumin and tetrahydroxycurcumin, which are all natural curcuminoids found in plants such as the curcuma species.

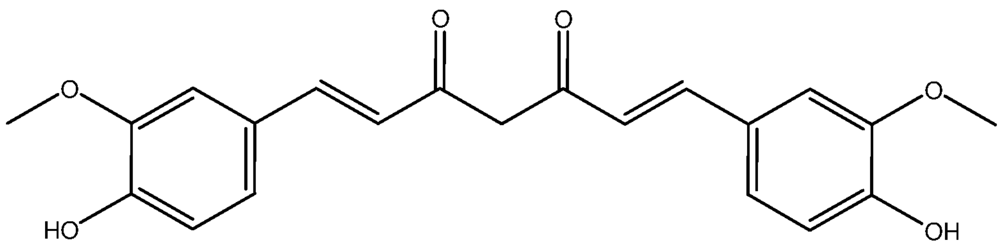

(1E,6E)-1,7-bis (4-hydroxy-3-methoxyphenyl)-6-heptadiene-3,5-dione (Curcumin)

The naturally occurring curcuminoids differ from one another in both the number and position of the methoxy groups, and all exhibit a keto-enol tautomerism in the midsection of the chromophore chain. These curcuminoids differ in their light absorbing and free radical scavenging properties. All of the naturally-occurring curcuminoids may serve as

efficient UV-blockers and/or stabilizers, in the context of embodiments of the present invention. For instance, some natural curcuminoid sources contain tetrahydroxycurcumin (THC), which is a colorless compound, which can be used as a UV stabilizer in applications where yellow color is not required or desired.

THC is a suitable curcuminoid compound, according to some embodiments of the present invention, in applications where yellowish tint is to be avoided.

Curcuminoid compounds can be extracted as naturally occurring substances or be prepared synthetically, and can be used as mixtures thereof or as isolated species. Curcuminoid compounds which are also encompassed by the present embodiments are disclosed in, for example, U.S. Patent No. 3,479,345, U.S. Patent Application Nos. 20030153512, 20060276536, 20070060644, 20070204412, 20080200478, 20100010232, 20100048901, 20100048901 and 20100087527, all of which are incorporated herein by reference as if fully set forth herein.

As discussed herein, substances that can leach (be extracted) out of a matrix may cause several adverse effects, such as harming the biological environment (tissue) surrounding the matrix, and reducing the effectiveness of the composition in, for example, blocking UV light. Hence, the characteristic behavior of the curcuminoid in polymeric or co-polymeric compositions can be referred to as low leachability (from the term "leachable") with respect to the matrix constituents and with respect to the curcuminoid compound, and can be defined in terms of comparison of concentrations before and after an extraction process.

Low leachability is significant when a substance is used in implantable devices, since in such devices, long-term performance is desired.

The process by which the incorporation of the curcuminoid compound is evaluated is also referred to as the extraction step of unreacted components and diluents (UCDs) from the cured composition, which is discussed in detail herein, and exemplified in the Example section that follows below.

According to some embodiments of the present invention, there is no perceivable difference in the concentration of the curcuminoid compound after the extraction process for removal of UCDs. Hence, according to some embodiments of the present invention, the polymeric or co-polymeric composition described herein as comprising a curcuminoid compound is such that the concentration of the curcuminoid compound in the composition does not decrease as a result of extraction step of UCDs, or decreases by no more than 0.0001 %, when subjected to an extraction process in an organic solvent, including also solvents which can readily dissolve the curcuminoid compound.

As discussed herein, a curcuminoid compound can be selected according to the desired light-absorption properties which are required from the composition.

According to some embodiments of the present invention, the curcuminoid can be selected such that the composition is substantially transparent to light at a wavelength ranging from about 400 to about 800 nm, and it can also be selected such that the composition is substantially opaque to light at a wavelength ranging from about 190 to about 440, or substantially opaque to light at a wavelength ranging from about 100 to about 400.

Such optical properties were demonstrated for compositions comprising curcumin, bisdemethoxycurcumin or monodemethoxycurcumin, as the curcuminoid compound.

According to some embodiments of the present invention, the curcuminoid can be selected such that the composition is substantially transparent to light at a wavelength ranging from about 490 to about 800 nm, and it can also be selected such that the composition is substantially opaque to light at a wavelength ranging from about 190 to about 440, or from about 100 to about 490. Such a composition is effective in reducing or essentially blocking the transmission of violet/blue light.

Such optical properties can be afforded when the curcuminoid compound is, for example, tetrahydroxycurcumin (THC).

As discussed above, UV-light is characterized by a wavelength ranging from 100-440 nm (some sources state that visible region starts from about 390 nm), violet is the color of the short-wavelength end of the human visible spectrum ranging approximately 380-450 nm, and blue light ranges 450-475 nm.

According to some embodiments of the present invention, the polymeric or co-polymeric composition as presented herein, which further comprises at least one curcuminoid compound as described herein is characterized by having UV-light blocking properties, wherein the UV-light is characterized by a wavelength ranging from 100 nm to 440 nm.

Some curcuminoid compounds exhibit yellow color which leads to absorption of visible blue and violet light. According to some embodiments of the present invention, the curcuminoid compound is such that it acts as a UV absorber as well as blue light blocker.

According to some embodiments, two different additives for radiation protection can be present in the composition, one for UV stabilization and another for visible blue light protection.

It is noted herein that the benefits of incorporating a curcuminoid compound in ophthalmic devices can apply also with lens device made from any polymeric or co-polymeric composition, as described herein.

The polymeric or co-polymeric composition for making lens device **100,** according to some embodiments of the present invention, can be prepared by conventional polymerization techniques as known in the art, which include mixing the monomers and optional additives, such as UV-blockers, into a homogeneous pre-polymerization mixture,

optional heating, degassing and adding additional ingredients, such a polymerization initiator such as a free radical polymerization initiator, and subjecting the mixture to polymerization conditions after casting the mixture into a mold.

These general processes allow adding any optional additive, such as the natural UV-blocker curcuminoid compound, at various steps of the process. For example, for preparing a co-polymeric composition as described herein, a mixture containing the five types of monomers is used. For preparing a polymeric or co-polymeric composition having a curcuminoid compound incorporated therein or thereon, a mixture containing aryl acrylate monomers as described herein is used.

Incorporation of the curcuminoid compound can be made while adding the curcuminoid compound to the pre-polymerization mixture or by contacting the composition with the curcuminoid compound and allowing it to be incorporated thereon.

Some processes of preparing the lens device further include post-polymerization steps such as chemical treatments, extractions and mechanical shaping.

However, some processes may be more suitable for forming lens devices made from particular compositions, as presented herein.

Hence, according to an aspect of embodiments of the present invention, there is provided a process of preparing the polymeric or co-polymeric composition presented herein, which is effected by:

admixing a pre-polymerization mixture containing the monomers presented hereinabove, as well as other optional constituents and additives and a free radical polymerization initiator;
optionally degassing the pre-polymerization mixture so as to remove any dissolved gasses which may interfere with optical clarity of the composition by forming vacuoles;
heating said pre-polymerization mixture while stirring;
optionally degassing the pre-polymerization mixture again so as remove volatile residues after heating;
optionally admixing an additional amount of the initiator into the pre-polymerization mixture so as to obtain a polymerization reaction mixture;
admixing a curing agent into the reaction mixture;
casting the reaction mixture into a mold;
exposing the reaction mixture in the mold to curing conditions, to thereby obtain the co-polymer composition presented herein; and
subjecting the co-polymeric composition to a multiple extraction so as to rid it from unreacted contaminants.

According to some embodiments of the present invention, the pre-polymerization mixture comprises the first, second, third, fourth, and fifth monomers in their appropriate ratios, as described herein for a hydrophobic co-polymeric composition.

According to some embodiments of the present invention, one of the additives can be a curcuminoid compound, as discussed hereinabove.

Optionally, heating the pre-polymerization mixture while stirring is performed at 40 °C until the viscosity of pre-polymerization mixture reaches an optimal level (120 cps at 25 °C). The viscosity measurements are obtained from the torque applied on the stirring device.

Once all the monomers and other components are mixed together for polymerization, the polymerization reaction may be initiated by adding a radical polymerization initiator in a conventional manner to obtain the polymeric or co-polymeric composition according to some embodiments of the present invention.

The choice of initiator also determines the kinetics of the polymerization reaction. As discussed hereinabove, the molecular weight of the composition imparts properties to the lens device, such as glistening, mechanical properties, refractive index and transmittance. Molecular weight is inversely proportional to the half power of the initiator concentration.

The initiator, also referred to herein as a catalyst, is typically employed to initiate the polymerization of the monomers and/or carry out the crosslinking or thermosetting of the polymeric or co-polymeric compositions formed of those monomers, as presented herein. Thus, according to some embodiments of the present invention, the co-polymeric composition present herein further includes an initiator (a catalyst).

According to some embodiments of the present invention, the polymerization reaction follows a free-radical propagation mechanism. As known in the art concerning conventional polymerization methods, the free-radical polymerization reaction may be initiated, for example, by free radical initiators, either thermally or photochemically. When using a thermally initiated free radical polymerization reaction, the method is typically effected by heating gradually from room temperature to an elevated temperature, such as 130 °C, and the temperature can be elevated stepwise and/or cycled. When the polymerization initiator is controlled photochemically, the polymerization reaction in initiated by irradiating the pre-polymerization mixture with electromagnetic radiation, such as microwave, ultraviolet light or radiation ($\gamma$ ray) after a radical polymerization initiator is added thereto. It is noted herein that two or more types of initiators may be combined

to arrive at a more controlled and completed polymerization reaction.

According to some embodiments of the present invention, the initiation step is effected at relatively low temperatures as a function of the choice of initiator. According to some embodiments of the present invention, the co-polymeric composition includes a low temperature dissociation initiator which keeps the formed lens device fixed in position in the mold by avoiding significant expansion or contraction. Using such initiators makes the use of fused silica molds redundant, which in turn reduces the need for complex UV curing of the composition. Hence, according to some embodiments of the present invention, the initiator is a low temperature dissociation initiator.

According to some embodiments of the present invention, non-limiting examples of the initiator include bedicetyl peroxydicarbonate, tert-butyl peroxypivalate, diisobutyryl peroxide, dimyristyl peroxydicarbonate, 1,1,3,3-tetramethylbutyl peroxypivalate, tert-butyl peroxyneoheptanoate, di(2-neodecanoylperoxy-isopropyl)benzene, cumylperoxy-neodecanoate, 1,1,3,3-tetramethylbutylperoxy-neodecanoate, t-butylperoxy-neodecanoate, t-butylperoxy-neoheptanoate and any combinations thereof.

According to other embodiments of the present invention, the catalyst is selected from the group consisting of dicetyl peroxydicarbonate (such as Perkadox® 24L by Akzo Nobel Polymer Chemicals, India) and tert-butyl peroxypivalate (such as LUPEROX® 554M75 by Arkema Inc. Philadelphia, PA, USA).

Non-limiting examples of a radical and/or thermal polymerization initiator include, for instance, azobisisobutyronitorile, azobisdimethylvaleronitrile, benzoyl peroxide, tert-butyl hydroperoxide, qumene hydroperoxide and the like, which can be used solely or in a combination use of two or more thereof.

Light-sensitive initiators (photopolymerization initiator) include, for a non-limiting example, photopolymerization initiators of benzoin compounds such as methyl orthobenzoyl benzoate, methyl benzoyl formate, benzoin methyl ether, benzoin ethyl ether, benzoin isopropyl ether, benzoin isobutyl ether and benzoin n-butyl ether, photopolymerization initiators of phenone compounds such as 2-hydroxy-2-methyl-1-phenylpropane- 1-one, p-isopropyl-$\alpha$-hydroxyisobutylphenone, p-tert-butyltrichloroacetophenone, 2,2-dimethoxy-2-phenylacetophenone, $\alpha$,$\alpha$-dichloro-4-phenoxyacetophenone, and N,N-tetraethyl-4,4-diaminobenzophenone, 1-hydroxycyclohexylphenylketone, 1 -phenyl-1,2-propanedione-2-(o-ethoxycarbonyl) oxime, photopolymerization initiators of thioxanthone such as 2-chlorothioxanthone and 2-methylthioxanthone, dibenzosuberone, 2-ethylanthraquinone, benzophenone acrylate, benzophenone, benzil and the like.

The amount of the above-mentioned polymerization initiator is typically not less than 0.002 percent by weight, more preferably 0.01 % by weight based on 100 % of the total weight of the composition. Alternatively, the concentration of the initiator is not more than 10 % by weight, or not more than 2 % of the total weight of the composition.

The optional addition of initiator (also referred to herein interchangeably as a catalyst) prior to the casting stage, is meant to boost the completion of the polymerization reaction. As discussed hereinabove, additional initiator is added when the viscosity of the reaction mixture reaches about 120 cps.

According to some embodiments of the present invention, the process includes first adding an amount equivalent to about 20-40 % of the total amount of the initiator in the first step of the process, and then adding about 60-80 % of the total weight of the initiator.

After mixing and adding the initiator, the mixture is transferred through a filter into the mold. The viscosity at which the mixture is filtered is about 120-130 cps. The ophthalmic device is formed in the mold from the mixture directly into its complete form, avoiding further machining which in turn avoids local burning and machining related stresses. After casting or pouring the mixture in between two halves of the mold, thermal or photo curing stimulus is applied. Thermal curing is performed at room temperature to 80 °C with several ramping steps.

According to some embodiments of the present invention, filtering the reaction mixture may also precede the casting stage.

Once the reaction mixture is cast in a mold, the curing step can start. Exposure to curing conditions typically includes elevating the temperature and/or exposing the reaction mixture to high energy radiation. High temperatures are typically about 80 °C, and the irradiation energy may range from 10 KJ/Kg to 50 KJ/Kg in order to further allow the co-polymeric composition to cure.

Curing agents and accelerators may also be employed in the formation of the co-polymeric composition according to some embodiments of the present invention. Various curing agents and accelerators are known and can be used in prescribed amounts or amounts experimentally found to be suitable. Typically, amounts of the curing agent, the curing agent accelerator or a combination thereof are between about 0.1% and about 8 % by weight of the total weight of the composition. Curing agents and accelerators can be used in various amounts, which will typically depend upon the monomers and polymers being employed, any ambient conditions (e.g., heat, light or otherwise) being used for curing and/or other factors.

Examples of suitable curing agents include UV photoinitatiators, peroxy catalysts (i.e., any catalyst including a peroxy group), oxide catalysts (i.e., any catalyst include an oxide group (e.g., a dioxide) or others known by the skilled artisan. One example of a peroxy catalyst is a tert-butyl peroxy-2-ethylhexanoate organic peroxide initiator, which is particularly suitable for thermal cure. One example of an oxide catalyst is 2,4,6-trimethylbenzoyldiphenylphosphine oxide, which is particularly suitable for blue light cure.

According to some embodiments of the present invention, the monomers, the initiator, the curing agent and optionally curing agent accelerator, a UV-blocker (radiation resistant compound), if present, and any other desired ingredients are combined together to form a master batch. The master batch is then exposed to an ambient stimulus, such as heat or light (e.g., blue light) which initiates polymerization and cross-linking reactions between the various monomers. The initiated master batch can be cast into molds (such as cast wafers) of desired geometry and can be secured in cure fixtures for forming the ophthalmic devices. It is advisable to add a crosslinking monomer in initial stage especially in the case where the polymerization reaction is performed at relatively low temperature.

The wafer molds are then typically cured through extended exposure to an ambient condition such as heat, light or both. For example, in one embodiment, the cast wafers are exposed to an elevated temperature (e.g., about 70 °C) for a first period of time (e.g., about 2 hours) and then ramped up to a second temperature (e.g., about 110 °C) for a second period of time (e.g., at least 10 minutes). In a second exemplary embodiment, the wafers are cured using blue light at a wavelength of about 405 nm to about 415 nm for a first period of time (e.g., about 3 hours) and then exposed to an elevated temperature (e.g., about 110 °C) for a second period of time (e.g., about one hour). Typically, the initiation, the curing or both are carried out in a low moisture (e.g., less than 1 ppm water) and low oxygen (less than 100 ppm) environment.

Alternatively, working at relatively low temperatures, from room temperature and up to 80 °C is advantageous, since it allows the polymerization and curing steps to complete post the casting stage (namely in the mold), without causing thermal distortion of the mold. This way a relatively low cost polypropylene mold can be used, instead of a costlier fused silica molds.

During the initial polymerization step it is possible to monitor the viscosity of the master batch mixture, for example by following the force applied to the mixing shaft.

Once the polymeric or co-polymeric composition is cured it can be cleansed from unreacted components and other leachables. The extraction of leachables may commence once the molded and cured composition is released from the mold. According to some embodiments of the present invention, the extraction is effected by sequential immersion of the polymeric or co-polymeric composition in a series of baths, each containing a different solvent or solution, going from hydrophobic to hydrophilic in the order of sequence, thereby extracting unreacted contaminants from the composition.

Following the description of the embodiments of the present invention, provides co-polymeric compositions and processes for preparing the same, which are highly suitable for manufacturing implantable and non-implantable ophthalmic and ocular devices including lens devices. Following the above-described description of the embodiments affords vacuole free, glistening free, internal reflection free and tack free ophthalmic and ocular devices, which meet the requirement of tensile strength, deformation recovery ability and mechanical, optical, biological and toxicological requirements set by widely accepted standards of the art.

It is noted that even after the post-curing step, which further pushes the polymerization reaction to completion, some impurities from unreacted monomers and other contaminants remains in the composition. These impurities are commonly referred to herein as unreacted components, diluents and other leachable impurities. Under the term leachable impurities are also included filter membrane residues, boiling impurities, solvent remnants and other process contaminants.

To remove unreacted components and diluents (UCDs) and other leachable impurities from the cured composition formed as a lens device, and affect clinical viability of the lens device, the process of preparing such devices typically includes an extraction step. If the leachable substances are not extracted from the device, they may make the device uncomfortable to wear or even present a medical hazard. As used herein, "leachable substance" includes UCDs and other substances which are not bound to or embedded in the polymer and may be extracted from the composition (the matrix), for example, by leaching with water or an organic solvent. As used herein, the term "treat" means to expose a cured object or device, made from the composition presented herein, to an aqueous and/or organic solution which may also include at least one leaching aid. Treating a cured polymeric composition to remove UCDs and monitoring traces thereof is demonstrated in the Examples section which follows below using a curcuminoid compound as a light-stabilizer, which is not part of the polymeric backbone and therefore required not to leach out of the cured composition.

As used herein, a "leaching aid" is any compound that if used in an effective amount in an aqueous or organic solution to treat an ophthalmic device, and can assist in obtaining a device with an adequate amount of removal of leachable substances.

According to embodiments of the present invention, the process of preparing the lens device from the co-polymeric composition presented herein may include a treatment of the cured co-polymeric composition. The treatment step can include exposing the cured composition to an aqueous and/or organic solution which constitutes or includes at least one leaching aid. In various embodiments, treatment can be accomplished, for example, via immersion of the device in a solution or exposing the device to a flow of solution or exposing the device to Soxhlet extraction. In various embodiments, treatment can also include, for example, one or more of heating the solution; stirring the solution; mechanical agitation or sonication of the solution; and increasing the level of leach aid in the solution to a level sufficient

to facilitate adequate removal of leachable substances from the device.

According to some embodiments of the present invention, an organic or aqueous solution may constitute a leaching aid. According to other embodiments of the present invention, leaching aids can also be combined with organic solvents to improve the rate of release. For example, in some embodiments, ophthalmic devices such as lenses can be subjected to a treatment exposing the lens devices to a leaching aid and a GC Mass Spectrometer can be used to measure the level of one or more leachable substances in the lens devices. The GC Mass Spectrometer can determine whether treatment with a particular leaching aid is effective to reduce an amount of particular leachable substances present in the lenses to a maximum threshold amount. Accordingly, in some embodiments, a GC Mass Spectrometer can be used to check for a maximum threshold of leachable substances of approximately 300 ppm. A minimum hydration treatment time period necessary to reduce the presence of such leachable substances to 300 ppm or less in specific lenses can be determined by the periodic measurements. In additional embodiments, other leachable substances, such as, for example, D3O or other diluents, can be measured to detect the presence of a maximum amount of approximately 60 ppm. Embodiments can also include setting a threshold amount of a particular leachable substance at the minimum detection level ascertainable by the testing equipment.

Examples of leaching aids, according to the present invention include, without limitations, alkanes, ketones (e.g. 2-butanone), amides, ethers (e.g. THF), alcohols (e.g. methanol), esters (e.g. ethyl acetate), aldehydes, nitrogen-containing cyclic compounds, toluene, water, ethoxylated alcohols or ethoxylated carboxylic acids, ethoxylated glucosides or sugars, optionally with attached $C_6$-$C_{14}$ carbon chains, polyalkylene oxides, sulfates, carboxylates or amine oxides of $C_6$-$C_{14}$ compounds. Examples include cocamidopropylamine oxide, $C_6$-$C_{14}$ fatty alcohol ethoxylated with ethylene oxides, sodium dodecyl sulfate, polyoxyethylene-2-ethyl hexyl ether, polypropylene glycol, polyethylene glycol monomethyl ether, ethoxylated methyl glucoside dioleate, and the sodium salt of n-octylsulfate, sodium salt of ethylhexyl sulfate.

By way of non-limiting examples, various implementations can include release and removal of leachable impurities that is accomplished by way of a batch process wherein devices are submerged in a solution contained in a fixed tank for a specified period of time or in a vertical process where devices are exposed to a continuous flow of a solution that includes at least one of a leach aid. In some embodiments, the solution can be heated with a heat exchanger or other heating apparatus to further facilitate leaching of the device. For example, heating can include raising the temperature of an aqueous or organic solution to the boiling point while a device is submerged in the heated solution. Other embodiments can include controlled cycling of the temperature of the solution. Some embodiments can also include the application of physical agitation to facilitate leach. For example, a strainer container holding the device can be vibrated or caused to move back and forth within a leaching solution. Other embodiments may include ultrasonic waves through the solution.

The choice of a leaching aid or solvent depends on the impurities present in the composition. Each solvent has a capability of extracting certain impurities with an overlapping range of chemical efficiency. For example, 2-butanone, THF, methanol and ethyl acetate are used sequentially on a same set of fully cured devices; which helps in removing all identified impurities step by step. Water miscible solvents are kept for last. A continuous soxhlet extraction may be employed which utilizes fresh solvent each time. Each soxhlet extracting phase may be sized to hold 500 lenses of 20 diopter. Maximum quantity varies with the power for given sized extractor. However extractors can be easily scaled up to meet the quantity requirement. Flow rate for the solvent varies according to extractor and according to cycle time. A holding time of 3 hours is kept for each solvent. For example, a drop rate of 100 ml/hr is kept for 300 ml extractor.

For example, freshly cured lens devices, prepared from the co-polymeric composition presented herein are kept in glass thimble, which is soaked in a bath of one leaching aid solution, and then exchanges its position to a bath holding the next solvent and so on; wherein each soak is maintained for 15-30 minutes. A vacuum tempering is performed thereafter in order to dry the devices at 110 °C and 0.1 mbar.

According to some embodiments of the present invention, post operation of reducing tack may be performed on a anterior surface or on both surface by processing lens by methods such as plasma treatment, surface fluorination, bulk fluorination, hydrophilic coating, irradiating with EB rays, high energy UV rays or by other energy intensive rays, use of internal wetting agents for selective migration and allied.

It should be noted herein that according to some embodiments of the present invention, the composition presented herein can be used to manufacture ophthalmic devices as described herein which are used also for drug delivery. In these embodiments, the non-leachability of the main additives and components (such as, for example, the curcuminoid compound discussed hereinbelow) is in effect, however, the drug which is delivered from the device to the surrounding tissue is in fact leachable, and can diffuse from the matrix (typically a hydrogel) to the physiological medium in which the device is situated and from there to the tissue to be treated.

A lens device as presented hereinabove, formed from the composition presented herein, can be manufactured by using one or two basic methods: molding into a final form without further machining, and shaping and molding followed by machining for reshaping and polishing. Hence, a device as described herein, made from a polymeric or co-polymeric composition as presented herein, can be made while preparing the polymeric or co-polymeric composition, or prepared

from a pre-formed polymeric or co-polymeric composition.

For example, multi-part molds can be used to fashion the composition presented hereinbelow into a useful article of a complex shape, such as lens device presented hereinabove or any ophthalmic lens. In the case of the lens device as presented herein, the multi-part molds can include for example, a first mold part with a convex or concave surface that corresponds to a back curve of an ophthalmic lens, and a second mold portion with a generally convex surface that corresponds to the complex structure of the front curve of the multifocal lens, which includes an inverse form of the plurality of concentric annular zones separated by slanted steps, as described herein. To prepare a lens using such mold portions, the uncured lens' composition is placed between the mold portions and subsequently cured. The lens' composition may then be cured, for example by exposure to either or both heat and light. The cured composition forms a lens body according to the dimensions and features of the mold portions. Following curing, traditional practice dictates that the mold portions are separated and the lens remains adhered to one of the mold portions, calling for a release process to detach the lens from the remaining mold part. In some embodiments, the process of manufacturing the lens body, the cured lens body having the plurality of concentric annular zones separated by slanted steps may be subjected to further polishing and/or shaping to achieve final desired form.

The formation of the ophthalmic device from a polymeric or co-polymeric composition as presented herein can also be effected by, for example, a computer-controllable manufacturing device. For example, lens device presented herein may be shaped into final form in two basic steps, wherein in the first step the crude lens is forged from the co-polymeric composition presented herein, and in the second step the plurality of concentric annular zones separated by slanted steps, as described herein, are formed in the cured crude lens device using a computer controllable manufacturing device.

Various embodiments and aspects of the present invention as delineated hereinabove and as claimed in the claims section below find experimental support in the following example.

## EXAMPLES

Reference is now made to the following example, which together with the above descriptions illustrates some embodiments of the invention in a non limiting fashion.

### *Exemplary lens device with 30 diffractive zones*

A prototype lens device was designed according to various exemplary embodiments of the present invention. The lens device included 30 zones and steps.

FIG. 7A illustrates the central (innermost) zone (referred to as zone 1), and FIG. 7B illustrates the peripheral (outermost) zone (referred to as zone 30). As shown, zone 1 has a step height $H$ = 1.83 microns and slope $s$ = 84°, and zone 30 has a step height $H$ = 0.09 microns and slope $s$ = 25°. Both zones exhibit diffractive for near vision and refractive power for far vision.

The values of step height $H$, step slope $s$ and step width $W_s$ for each of the 30 steps is summarized in Table 1, below.

Table 1

| Zone No. | Step height [μm] | Step slope [degrees] | Step width [μm] |
|---|---|---|---|
| 1 | 1.83 | 84 | 0.19 |
| 2 | 1.77 | 84 | 0.19 |
| 3 | 1.71 | 84 | 0.18 |
| 4 | 1.64 | 83 | 0.19 |
| 5 | 1.57 | 83 | 0.19 |
| 6 | 1.51 | 83 | 0.19 |
| 7 | 1.45 | 83 | 0.19 |
| 8 | 1.40 | 83 | 0.18 |
| 9 | 1.32 | 82 | 0.18 |
| 10 | 1.26 | 81 | 0.19 |
| 11 | 1.19 | 81 | 0.18 |

(continued)

| Zone No. | Step height [μm] | Step slope [degrees] | Step width [μm] |
|---|---|---|---|
| 12 | 1.16 | 81 | 0.19 |
| 13 | 1.09 | 80 | 0.19 |
| 14 | 1.01 | 80 | 0.18 |
| 15 | 0.94 | 79 | 0.19 |
| 16 | 0.90 | 79 | 0.18 |
| 17 | 0.90 | 79 | 0.18 |
| 18 | 0.82 | 77 | 0.19 |
| 19 | 0.77 | 76 | 0.19 |
| 20 | 0.70 | 75 | 0.19 |
| 21 | 0.64 | 73 | 0.19 |
| 22 | 0.60 | 72 | 0.19 |
| 23 | 0.51 | 70 | 0.19 |
| 24 | 0.46 | 68 | 0.19 |
| 25 | 0.41 | 65 | 0.19 |
| 26 | 0.36 | 62 | 0.19 |
| 27 | 0.30 | 58 | 0.19 |
| 28 | 0.20 | 46 | 0.19 |
| 29 | 0.14 | 36 | 0.19 |
| 30 | 0.09 | 25 | 0.19 |

***Exemplary lens device with 26 diffractive zones***

A prototype lens device was designed according to various exemplary embodiments of the present invention. The lens device included 26 zones and steps, with a variable step height of from 1.6364 μm at zone 1 to 0.72 μm at zone 26.

The radii and widths of the zones are listed in Table 2, below.

Table 2

| Zone No. | Zone radius [mm] | Zone width [mm] |
|---|---|---|
| 1 | 0.58704 | 0.58704 |
| 2 | 0.83027 | 0.24323 |
| 3 | 1.01696 | 0.18669 |
| 4 | 1.1743966 | 0.15743 |
| 5 | 1.3131323 | 0.13874 |
| 6 | 1.4385925 | 0.12546 |
| 7 | 1.55399 | 0.1154 |
| 8 | 1.66144 | 0.10744 |
| 9 | 1.76238 | 0.10094 |
| 10 | 1.85788 | 0.09550 |
| 11 | 1.94873 | 0.09085 |
| 12 | 2.03556 | 0.08683 |

(continued)

| Zone No. | Zone radius [mm] | Zone width [mm] |
|---|---|---|
| 13 | 2.11887 | 0.08331 |
| 14 | 2.19905 | 0.08018 |
| 15 | 2.27644 | 0.07738 |
| 16 | 2.35130 | 0.07487 |
| 17 | 2.42388 | 0.07258 |
| 18 | 2.49437 | 0.07049 |
| 19 | 2.56296 | 0.068579 |
| 20 | 2.62977 | 0.066815 |
| 21 | 2.69495 | 0.065182 |
| 22 | 2.75861 | 0.063664 |
| 23 | 2.82087 | 0.062250 |
| 24 | 2.88179 | 0.060927 |
| 25 | 2.94148 | 0.059686 |
| 26 | 2.99999 | 0.058519 |

FIG. 8A is a schematic illustration of the shape of the diffractive pattern **13,** and FIG. 8B is a schematic illustration of the diffractive pattern **13** once formed on the aspheric side **12a** of the lens device.

A profile view of the toric side **12b** of the lens device is schematically illustrated in FIG. 8C.

It is to be understood that a lens device having number of zones which differs from 26 and 30 is not excluded from the scope of the present invention. Furthermore, the radius and width values in Tables 1 and 2 above are not to be considered is limiting. For example, the radius and/or width value of a particular zone can be different from the value cited in Table 1 or Table 2, by up to P%, where P equals about 1 or about 2 or about 3 or about 4 or about 5 or about 10 or about 20.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A phakic lens device (100), being structurally adapted to be positioned in the posterior chamber (204) of the eye and comprising:

    a generally circular optical section (102) **characterized by** at least one optical power;
    two flat haptic structures (104) at radially opposite sides of said optical part,
    a vaulted section (108) connecting said optical section (102) and said haptic structures (104); **characterised in that** each of said flat haptic structures (104) having at lest three outwardly protruding pads for fixating said haptic structures (104) in the ciliary sulcus.

2. The device (100) according to claim 1, wherein said vaulted section (108) comprises at least one opening (110) for allowing flow of liquid, through said vaulted section (108), between the posterior chamber (204) and the anterior chamber (200) of the eye.

3. The device (100) according to any of claims 1 and 2, wherein said optical section (102) comprises at least one opening (116) for allowing flow of liquid, through said optical section (102), between the posterior chamber (204) and the anterior chamber (200) of the eye.

4. The device (100) according to any of claims 1-3, further comprising a transition zone (118) between said optical

section (102) and said vaulted section (108), wherein said transition zone (118) has at least one opening (120) for allowing flow of liquid, through said transition zone (118), between the posterior chamber (204) and the anterior chamber (200) of the eye.

5. The device (100) according to any of claims 1-4, being structurally adapted to be positioned below the iris (206) level by its entirety.

6. The device (100) according to any of claims 1-5, wherein said at least one opening (110) of said vaulted section (108) has an aspect ratio of less than 2.

7. The device (100) according to any of claims 1-5, wherein said at least one opening (110) of said vaulted section (108) is elongated.

8. The device (100) according to any of claims 1-5, wherein said at least one opening (110) of said vaulted section (108) is curved, with a convex side of said curve facing said optical section (102) and a concave side of the curve facing said haptic structures (104).

9. The device (100) according to any of claims 1-8, wherein said at least one optical power is refractive.

10. The device (100) according to any of claims 1-5, wherein said at least one optical power is diffractive.

11. The device (100) according to any of claims 1-10, wherein said optical section (102) is monofocal.

12. The device (100) according to any of claims 1-10, wherein said optical section (102) is multifocal.

13. The device (100) according to any of claims 1-12, wherein a first optical side (12a) of said optical section (102) has an aspheric profile and a diffractive pattern formed thereon, and an opposite optical side (12b) of said optical section (102) has a toric profile and is devoid of any diffractive pattern.

14. The device (100) according to claim 13, wherein said toric profile is devoid of spherical aberration.

15. The device (100) according to any of claims 1-14, wherein said optical section (102) comprises a diffractive pattern (13) having a plurality of concentric annular zones (14) having surfaces separated by slanted steps (16) having surfaces, wherein said surfaces of said concentric zones (14) effect both diffraction and refraction of incident light, while said surfaces of said steps (16) are substantially devoid of any diffractive or refractive power.

## Patentansprüche

1. Phakische Linsenvorrichtung (100), die strukturell angepasst ist, um in der hinteren Kammer (204) des Auges positioniert zu werden, und die aufweist:

   einen im Allgemeinen kreisförmigen optischen Abschnitt (102), der durch mindestens eine optische Leistung gekennzeichnet ist;
   zwei flache haptische Strukturen (104) an radial gegenüberliegenden Seiten des optischen Teils,
   einen gewölbten Abschnitt (108), der den optischen Abschnitt (102) und die haptischen Strukturen (104) verbindet; **dadurch gekennzeichnet, dass** jede der flachen haptischen Strukturen (104) mindestens drei nach außen vorstehende Pads zum Fixieren der haptischen Strukturen (104) in dem Ziliarsulkus aufweist.

2. Vorrichtung (100) nach Anspruch 1, wobei der gewölbte Abschnitt (108) mindestens eine Öffnung (110) aufweist, um einen Strom von Flüssigkeit durch den gewölbten Abschnitt (108) zwischen der hinteren Kammer (204) und der vorderen Kammer (200) des Auges zu ermöglichen.

3. Vorrichtung (100) nach einem der Ansprüche 1 und 2, wobei der optische Abschnitt (102) mindestens eine Öffnung (116) aufweist, um einen Strom von Flüssigkeit durch den optischen Abschnitt (102) zwischen der hinteren Kammer (204) und der vorderen Kammer (200) des Auges zu ermöglichen.

4. Vorrichtung (100) nach einem der Ansprüche 1-3, die ferner eine Übergangszone (118) zwischen dem optischen

Abschnitt (102) und dem gewölbten Abschnitt (108) aufweist, wobei die Übergangszone (118) mindestens eine Öffnung (120) aufweist, um einen Strom von Flüssigkeit durch die Übergangszone (118) zwischen der hinteren Kammer (204) und der vorderen Kammer (200) des Auges zu ermöglichen.

**5.** Vorrichtung (100) nach einem der Ansprüche 1-4, die strukturell angepasst ist, um vollständig unterhalb der Iris (206) positioniert zu werden.

**6.** Vorrichtung (100) nach einem der Ansprüche 1-5, wobei die mindestens eine Öffnung (110) des gewölbten Abschnitts (108) ein Seitenverhältnis von weniger als 2 aufweist.

**7.** Vorrichtung (100) nach einem der Ansprüche 1-5, wobei die mindestens eine Öffnung (110) des gewölbten Abschnitts (108) verlängert ist.

**8.** Vorrichtung (100) nach einem der Ansprüche 1-5, wobei die mindestens eine Öffnung (110) des gewölbten Abschnitts (108) gekrümmt ist, wobei eine konvexe Seite der Kurve dem optischen Abschnitt (102) zugewandt ist und eine konkave Seite der Kurve den haptischen Strukturen (104) zugewandt ist.

**9.** Vorrichtung (100) nach einem der Ansprüche 1-8, wobei die mindestens eine optische Leistung brechend ist.

**10.** Vorrichtung (100) nach einem der Ansprüche 1-5, wobei die mindestens eine optische Leistung beugend ist.

**11.** Vorrichtung (100) nach einem der Ansprüche 1-10, wobei der optische Abschnitt (102) monofokal ist.

**12.** Vorrichtung (100) nach einem der Ansprüche 1-10, wobei der optische Abschnitt (102) multifokal ist.

**13.** Vorrichtung (100) nach einem der Ansprüche 1-12, wobei eine erste optische Seite (12a) des optischen Abschnitts (102) ein asphärisches Profil und ein darauf ausgebildetes Beugungsmuster aufweist, und eine gegenüberliegende optische Seite (12b) des optischen Abschnitts (102) ein torisches Profil aufweist und kein Beugungsmuster aufweist.

**14.** Vorrichtung (100) nach Anspruch 13, wobei das torische Profil frei von sphärischer Aberration ist.

**15.** Vorrichtung (100) nach einem der Ansprüche 1-14, wobei der optische Abschnitt (102) ein Beugungsmuster (13) aufweist, das mehrere konzentrische ringförmige Zonen (14) mit Flächen hat, die durch geneigte Stufen (16) getrennt sind, die Flächen haben, wobei die Flächen der konzentrischen Zonen (14) sowohl eine Beugung als auch eine Brechung des einfallenden Lichts bewirken, während die Flächen der Stufen (16) im Wesentlichen weder eine beugende noch eine brechende Leistung aufweisen.

**Revendications**

**1.** Dispositif de lentille phakique (100), structurellement adapté pour être positionné dans la chambre postérieure (204) de l'oeil et comprenant :

une section optique généralement circulaire (102) **caractérisée par** au moins une puissance optique ;
deux structures haptiques plates (104) sur des côtés radialement opposés de ladite partie optique,
une section bombée (108) raccordant ladite section optique (102) et lesdites structures haptiques (104) ; **caractérisé en ce que** chacune desdites structures haptiques plates (104) a au moins trois tampons faisant saillie vers l'extérieur pour fixer lesdites structures haptiques (104) dans le sillon ciliaire.

**2.** Dispositif (100) selon la revendication 1, dans lequel ladite section bombée (108) comprend au moins une ouverture (110) pour permettre l'écoulement de liquide, à travers ladite section bombée (108), entre la chambre postérieure (204) et la chambre antérieure (200) de l'oeil.

**3.** Dispositif (100) selon l'une quelconque des revendications 1 et 2, dans lequel ladite section optique (102) comprend au moins une ouverture (116) pour permettre l'écoulement de liquide, à travers ladite section optique (102), entre la chambre postérieure (204) et la chambre antérieure (200) de l'oeil.

**4.** Dispositif (100) selon l'une quelconque des revendications 1 à 3, comprenant en outre une zone de transition (118)

entre ladite section optique (102) et ladite section bombée (108), dans lequel ladite zone de transition (118) a au moins une ouverture (120) pour permettre l'écoulement de liquide, à travers ladite zone de transition (118), entre la chambre postérieure (204) et la chambre antérieure (200) de l'oeil.

**5.** Dispositif (100) selon l'une quelconque des revendications 1 à 4, structurellement adapté pour être positionné en dessous du niveau de l'iris (206) dans sa totalité.

**6.** Dispositif (100) selon l'une quelconque des revendications 1 à 5, dans lequel ladite au moins une ouverture (110) de ladite section bombée (108) a un rapport d'aspect inférieur à 2.

**7.** Dispositif (100) selon l'une quelconque des revendications 1 à 5, dans lequel ladite au moins une ouverture (110) de ladite section bombée (108) est allongée.

**8.** Dispositif (100) selon l'une quelconque des revendications 1 à 5, dans lequel ladite au moins une ouverture (110) de ladite section bombée (108) est courbée, un côté convexe de ladite courbe faisant face à ladite section optique (102) et un côté concave de la courbe faisant face auxdites structures haptiques (104).

**9.** Dispositif (100) selon l'une quelconque des revendications 1 à 8, dans lequel ladite au moins une puissance optique est réfractive.

**10.** Dispositif (100) selon l'une quelconque des revendications 1 à 5, dans lequel ladite au moins une puissance optique est diffractive.

**11.** Dispositif (100) selon l'une quelconque des revendications 1 à 10, dans lequel ladite section optique (102) est monofocale.

**12.** Dispositif (100) selon l'une quelconque des revendications 1 à 10, dans lequel ladite section optique (102) est multifocale.

**13.** Dispositif (100) selon l'une quelconque des revendications 1 à 12, dans lequel un premier côté optique (12a) de ladite section optique (102) a un profil asphérique et un motif diffractif formé sur celle-ci, et un côté optique opposé (12b) de ladite section optique (102) a un profil torique et est dépourvu de tout motif diffractif.

**14.** Dispositif (100) selon la revendication 13, dans lequel ledit profil torique est dépourvu d'aberration sphérique.

**15.** Dispositif (100) selon l'une quelconque des revendications 1 à 14, dans lequel ladite section optique (102) comprend un motif diffractif (13) ayant une pluralité de zones annulaires concentriques (14) ayant des surfaces séparées par des étages inclinés (16) ayant des surfaces, dans lequel lesdites surfaces desdites zones concentriques (14) effectuent à la fois la diffraction et la réfraction de lumière incidente, alors que lesdites surfaces desdits étages (16) sont sensiblement dépourvues de toute puissance diffractive ou réfractive.

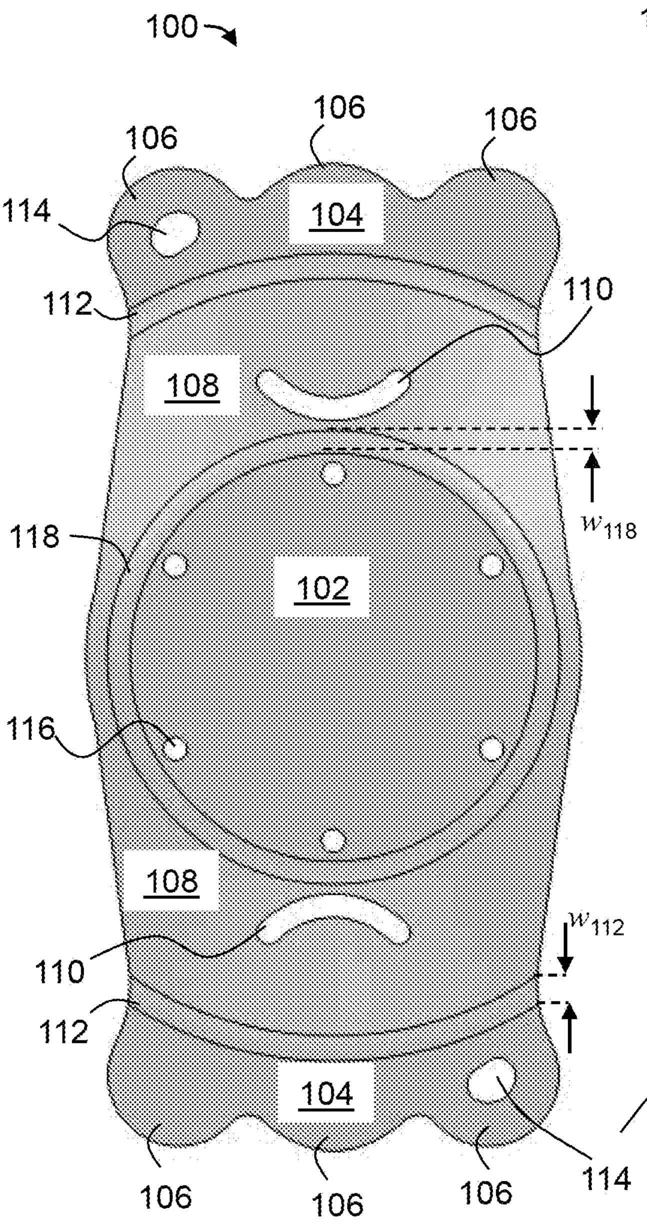
100
106
106
106
114
104
112
110
108
$w_{118}$
118
102
116
108
$w_{112}$
110
112
104
114
106
106
106

FIG. 1A

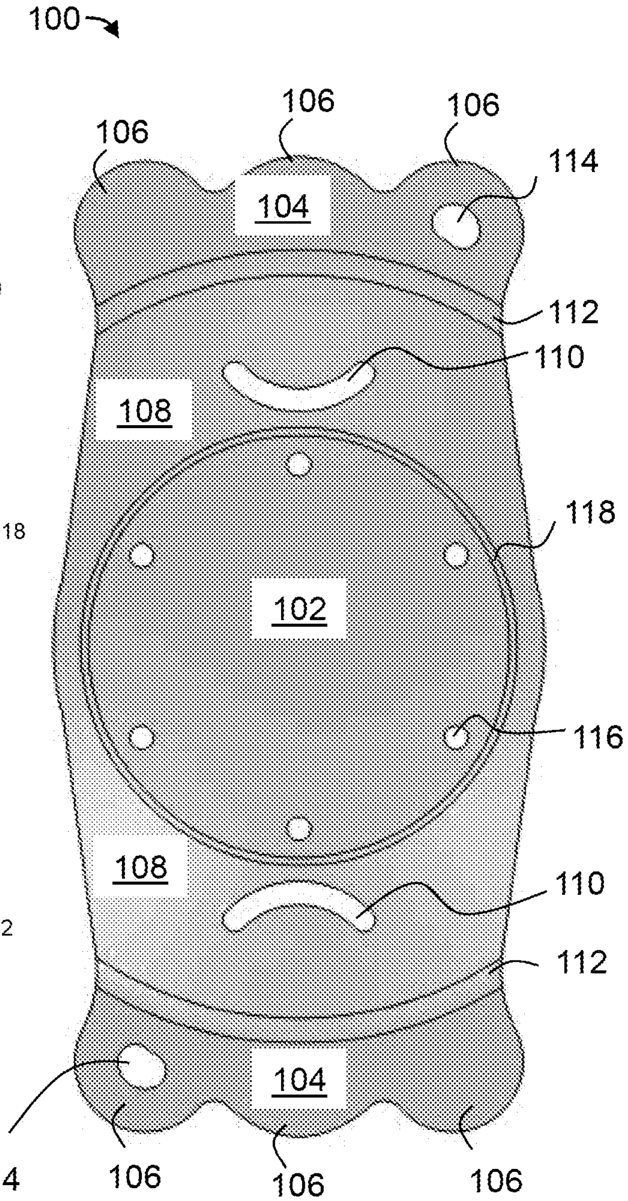
100
106
106
106
114
104
112
110
108
118
102
116
108
110
112
104
106
106
106

FIG. 1B

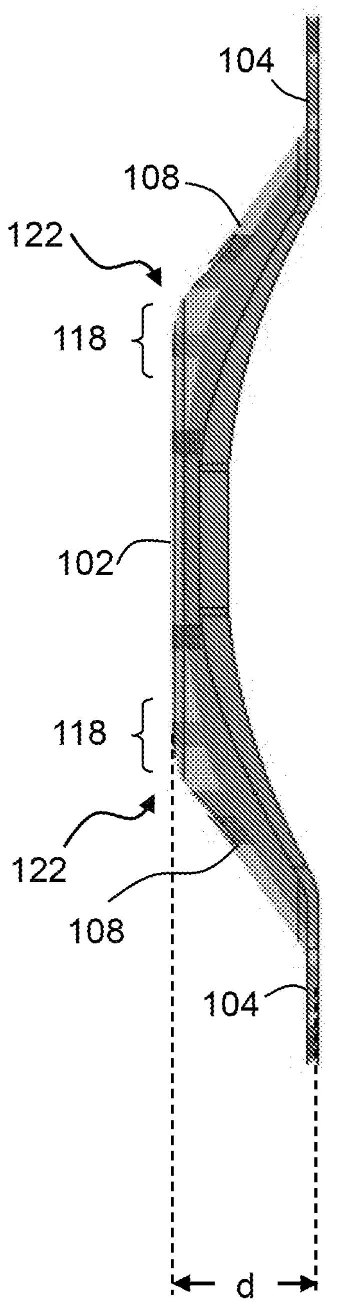
104
108
122
118
102
118
122
108
104
d

FIG. 1C

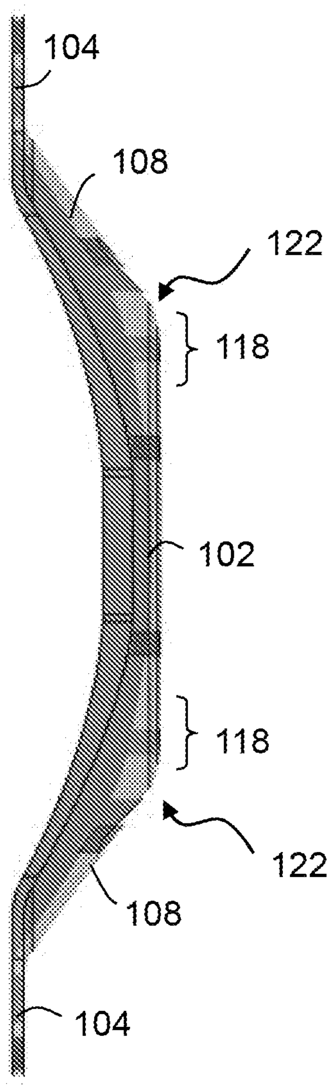
104
108
122
118
102
118
122
108
104

FIG. 1D

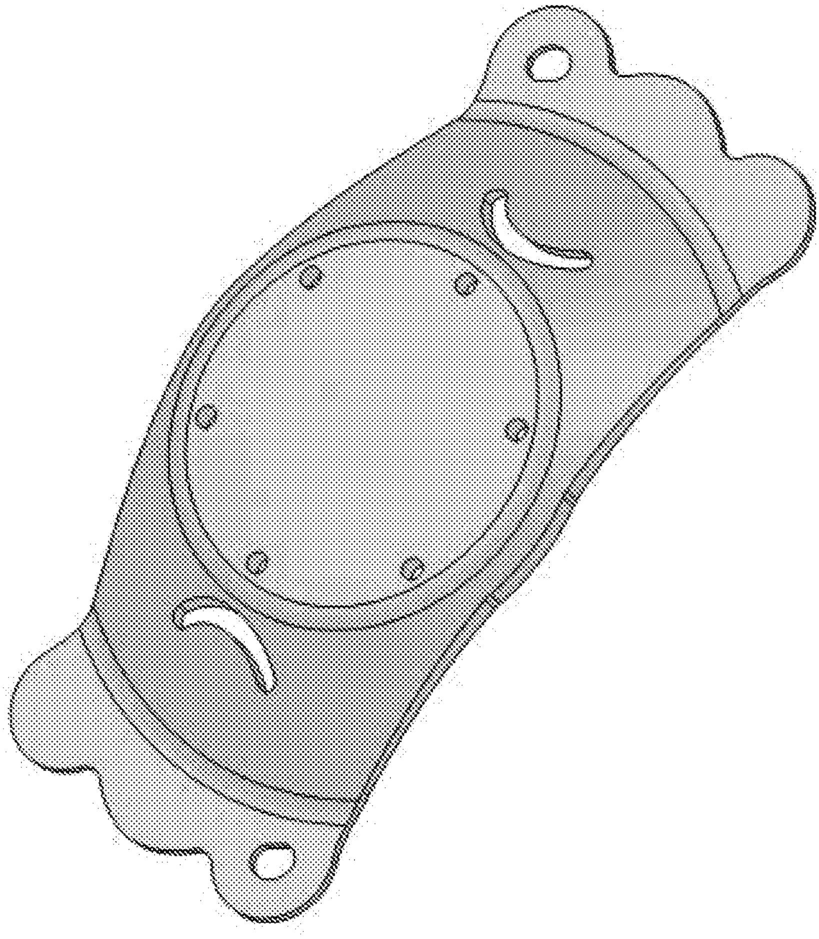

FIG. 1E

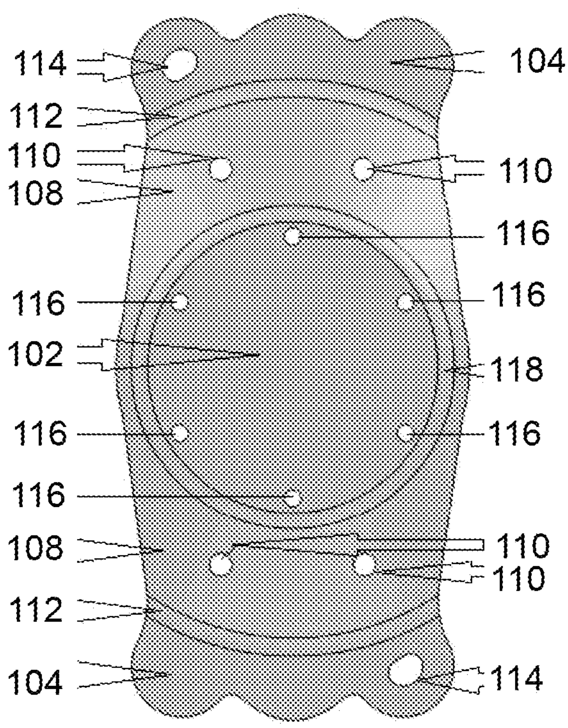
114
104
112
110
110
108
116
116
116
102
118
116
116
116
108
110
110
112
104
114

FIG. 2A

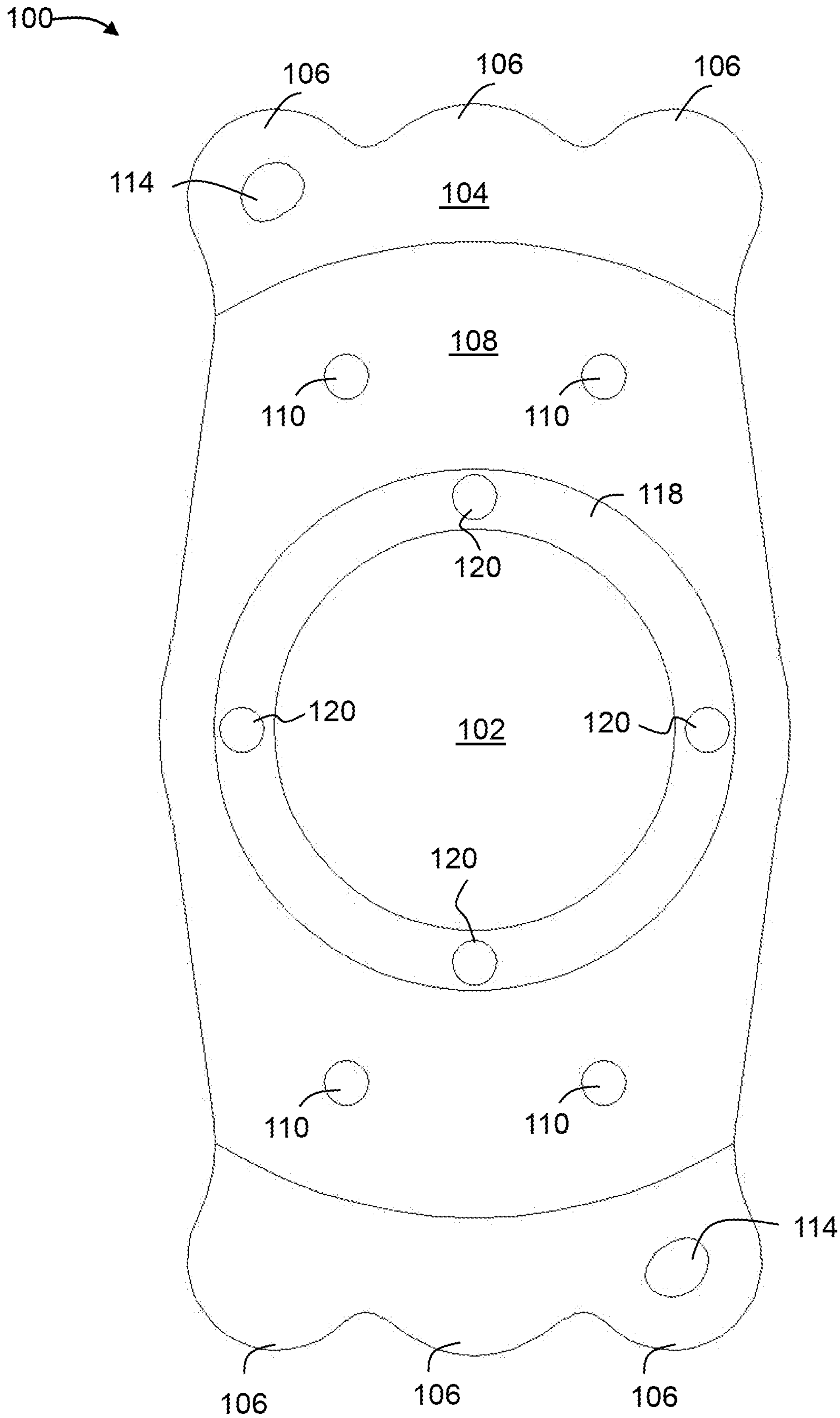
100
106
106
106
114
104
108
110
110
118
120
120
102
120
120
110
110
114
106
106
106

FIG. 2B

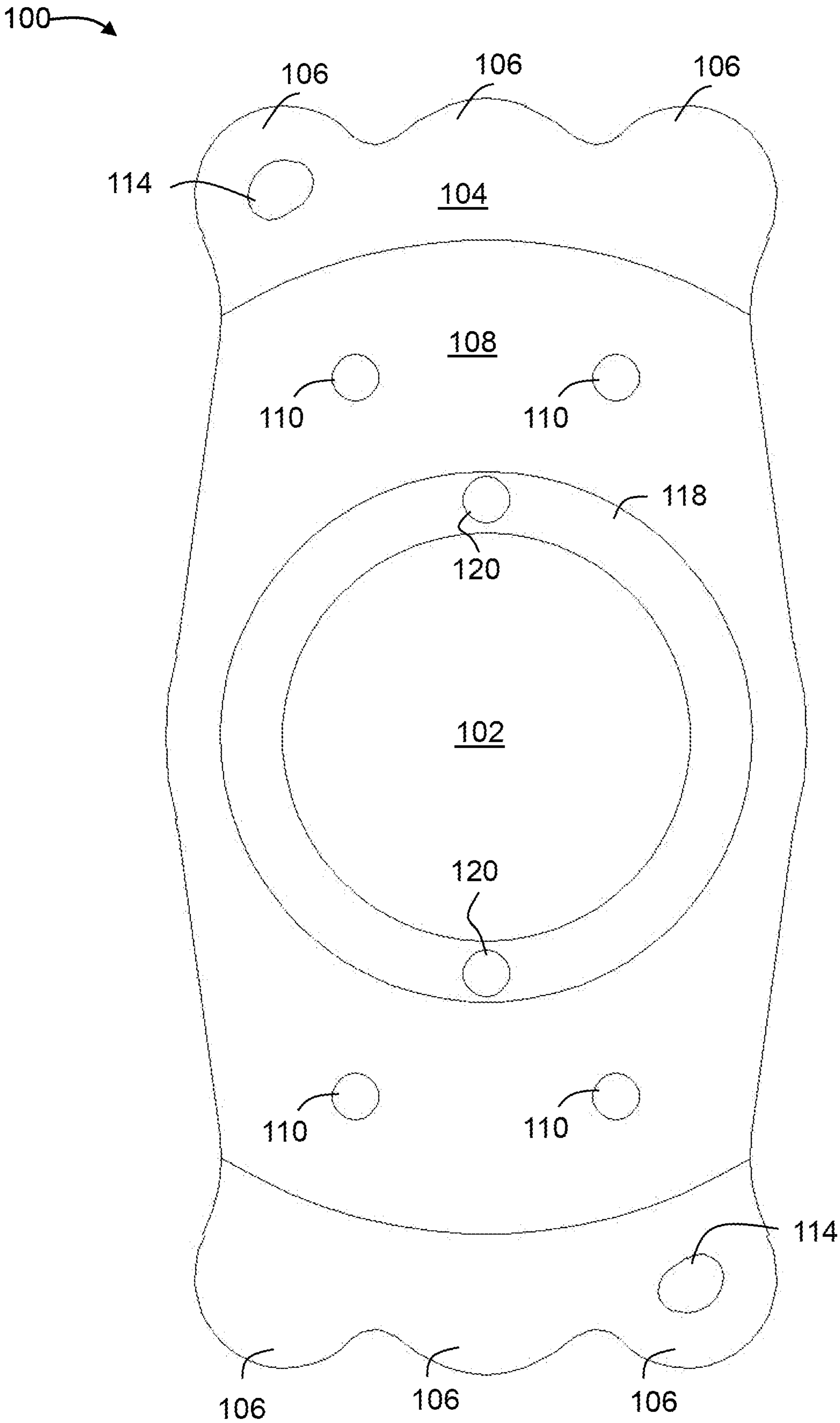
100
106
106
106
114
104
108
110
110
118
120
102
120
110
110
114
106
106
106

FIG. 2C

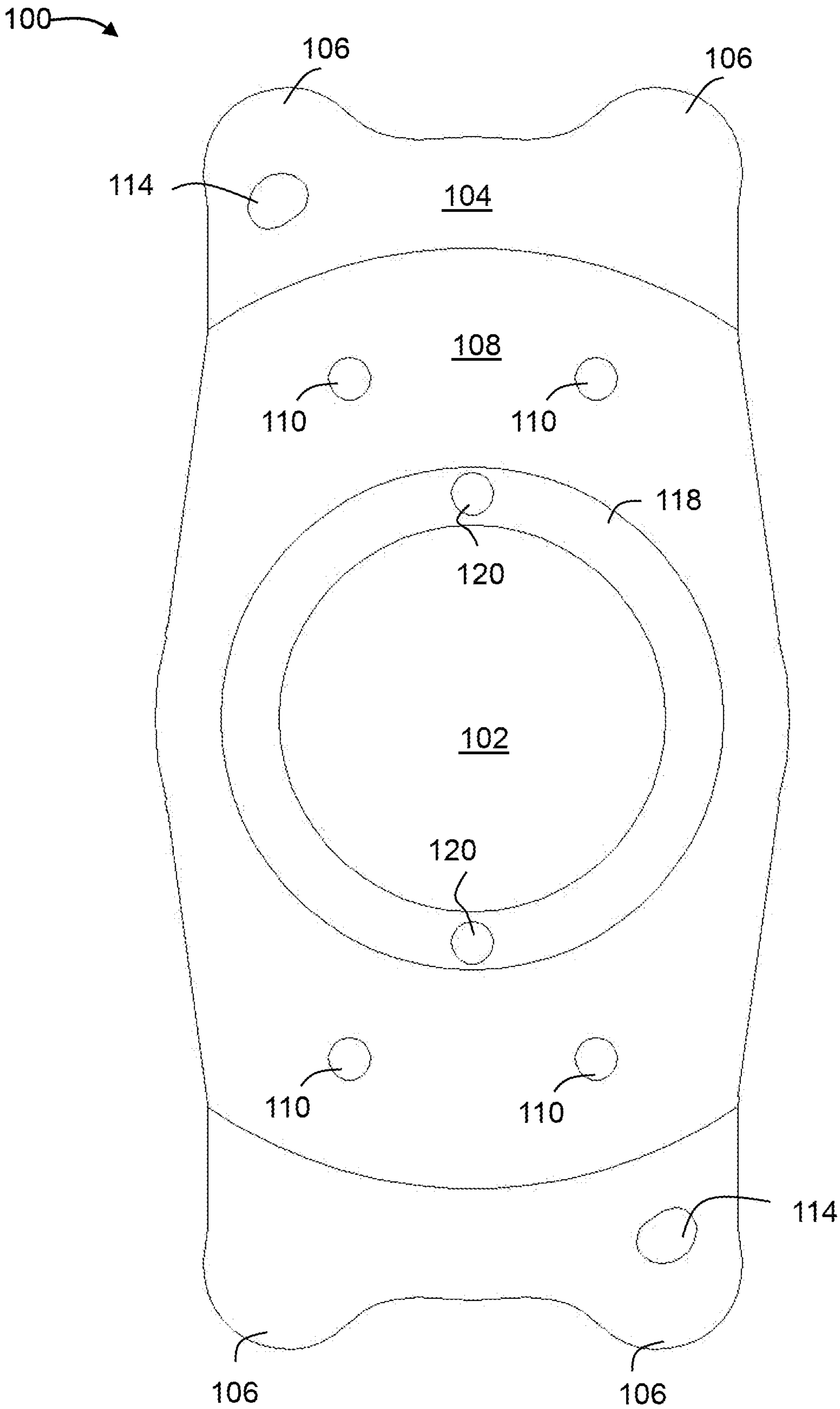
100
106
106
114
104
108
110
110
118
120
102
120
110
110
114
106
106

FIG. 2D

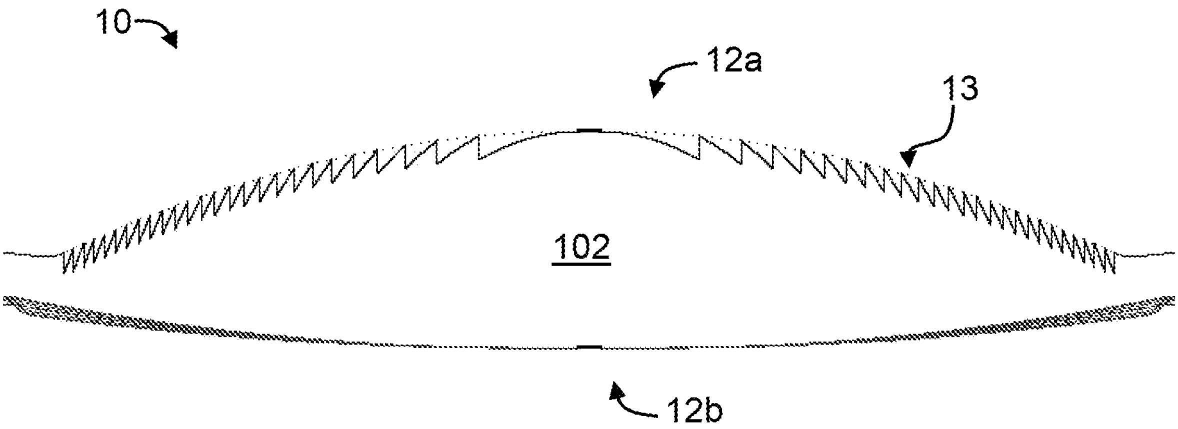


FIG. 3

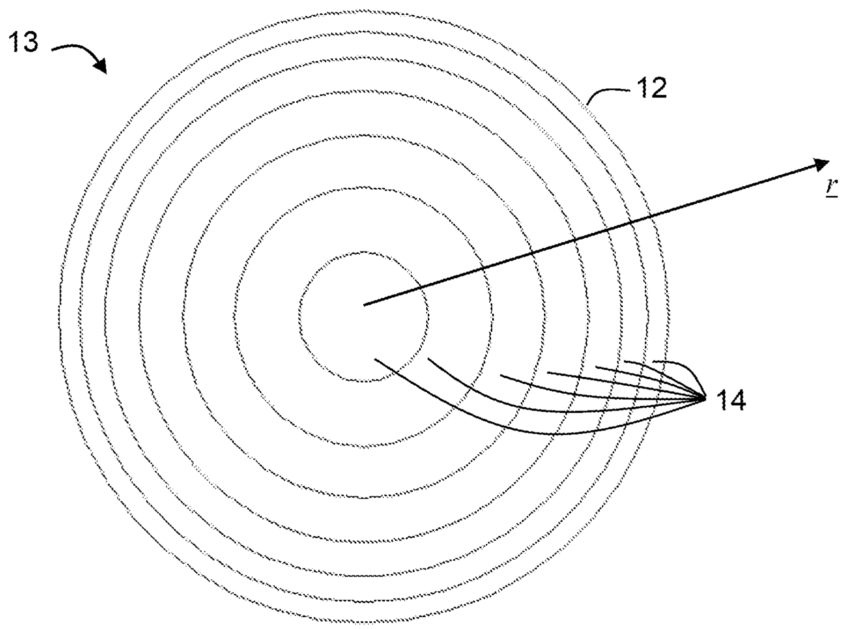


FIG. 4A

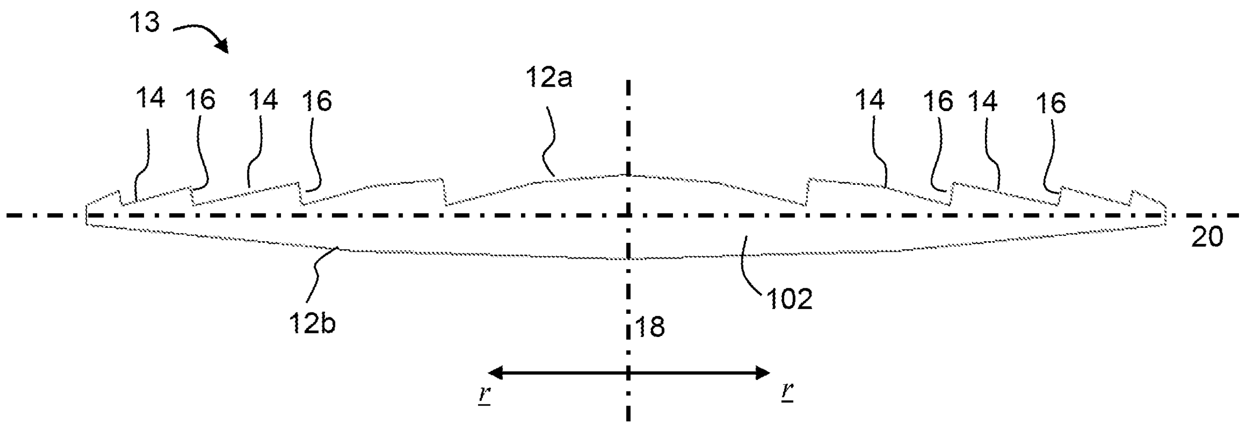


FIG. 4B

FIG. 5A

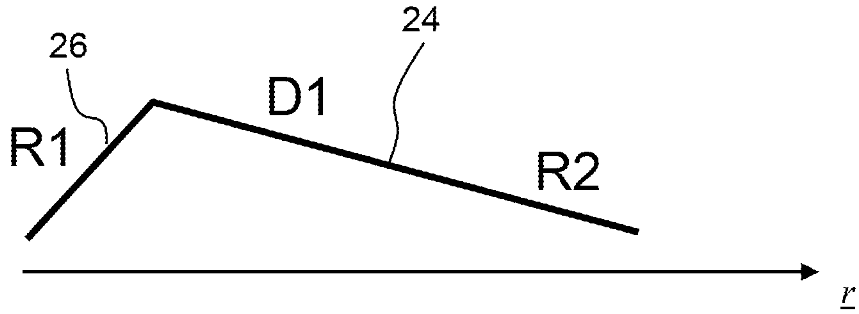


FIG. 5B

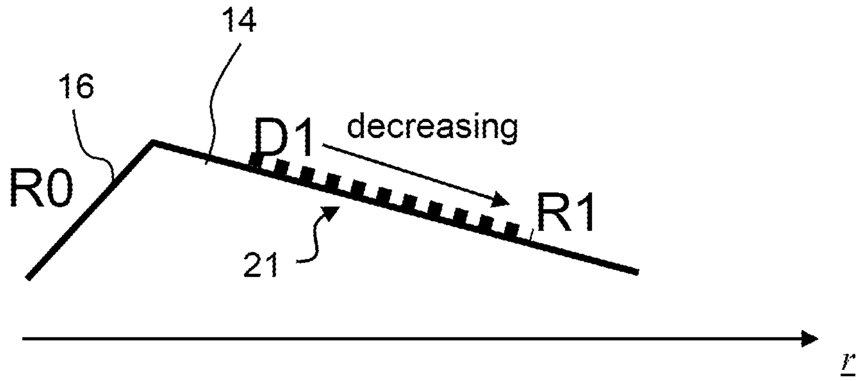


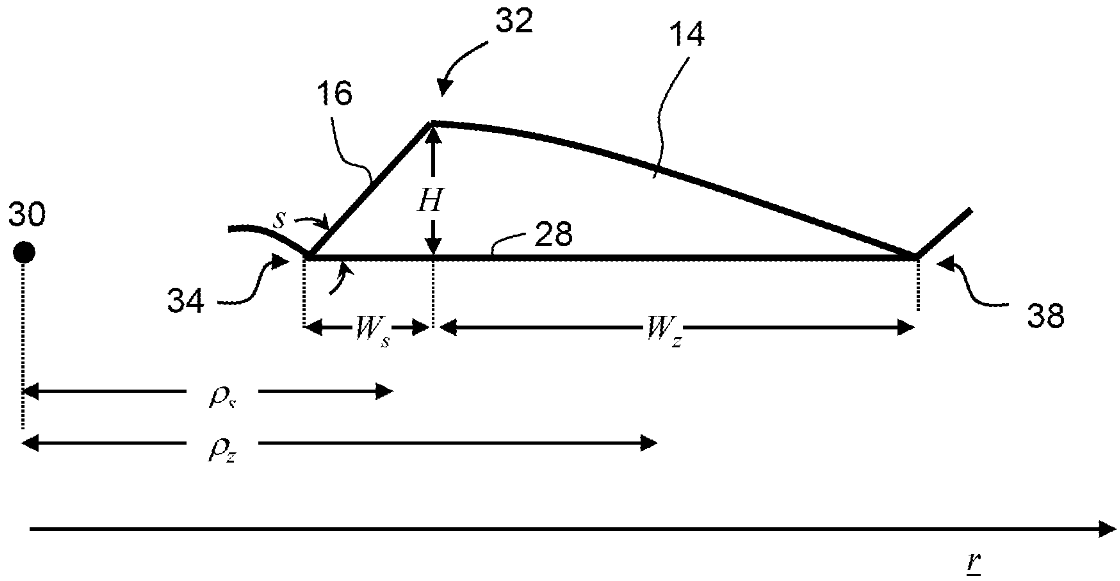


FIG. 6

FIG. 7A

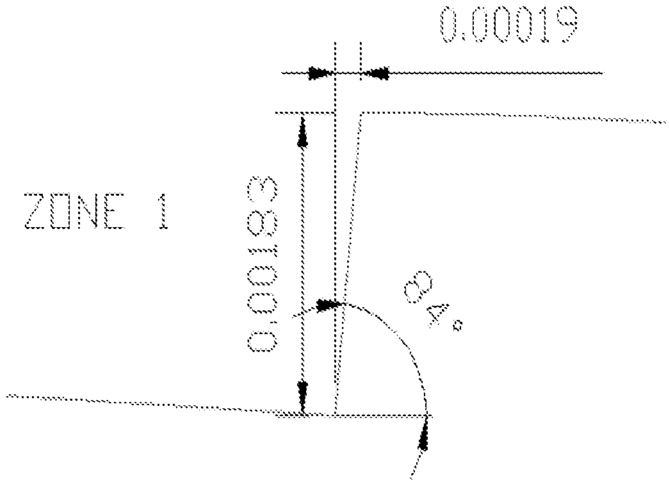


FIG. 7B

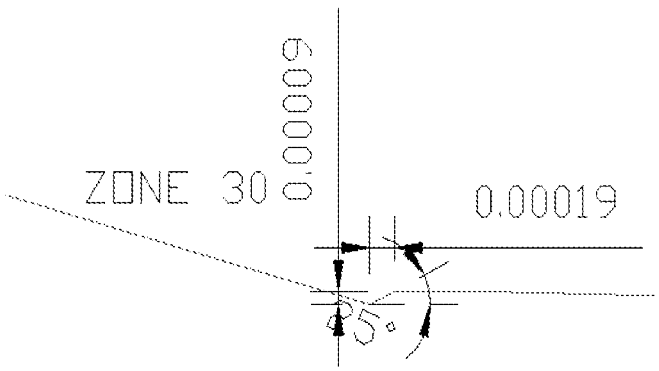

FIG. 8A

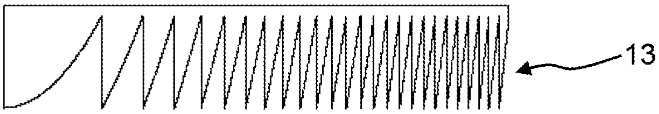


FIG. 8B

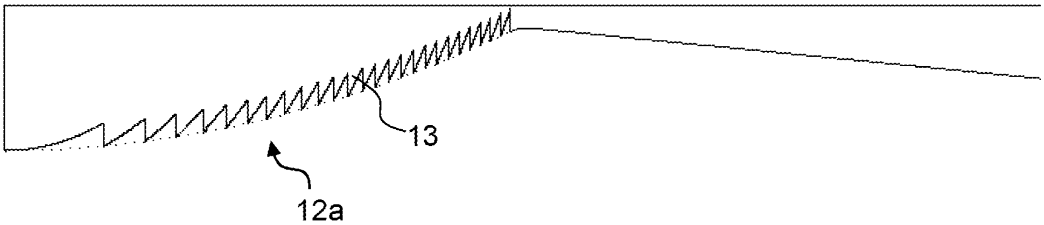


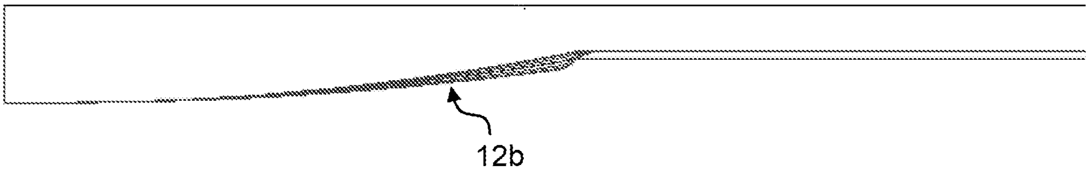


FIG. 8C

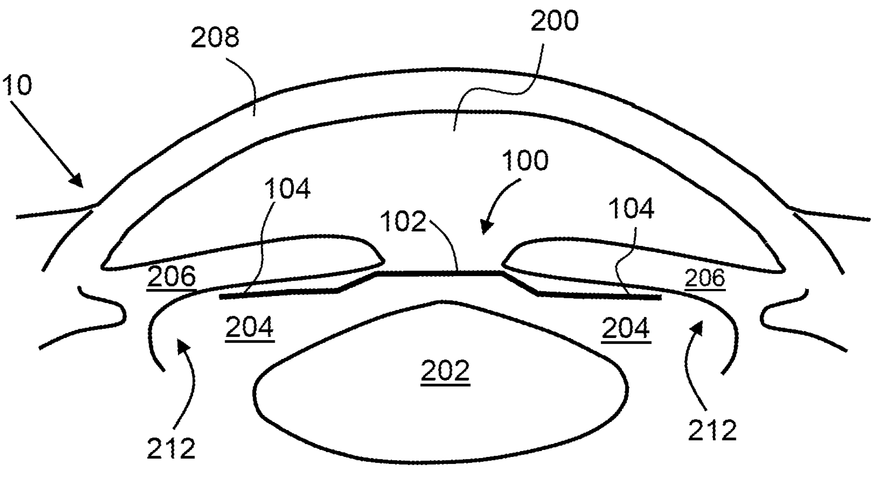


FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4585456 A, Blackmore
- US 5258025 A
- US 5078742 A
- US 4769035 A
- US 6015435 A
- US 6106553 A
- WO 1995028897 A
- WO 2002003891 A
- US 20050149184 A
- US 5290892 A
- US 4304895 A
- US 4528311 A
- US 7691918 B
- US 3479345 A
- US 20030153512 A
- US 20060276536 A
- US 20070060644 A
- US 20070204412 A
- US 20080200478 A
- US 20100010232 A
- US 20100048901 A
- US 20100087527 A